# EUROPEAN PATENT SPECIFICATION

(11) **EP 3 834 877 B1**
(45) Date of publication and mention of the grant of the patent: **14.06.2023**
(21) Application number: 19847692.1
(22) Date of filing: 22.07.2019
(51) Int. Cl.: A61M 25/02

(54) **CATHETER POSITIONING DEVICE**
KATHETERPOSITIONIERUNGSVORRICHTUNG
DISPOSITIF DE POSE DE CATHÉTER

(30) Priority: 07.08.2018 CN 201810890819
(43) Date of publication of application: 16.06.2021
(73) Proprietor: Shanghai Corinline Medical Technology Co., Ltd, Shanghai 200433 (CN)
(72) Inventor: XIAO, Liang, Shanghai 200433 (CN); GAO, Xiang, Shanghai 201399 (CN); WANG, Zhaoqin, Shanghai 201399 (CN)
(74) Representative: Viering, Jentschura & Partner mbB Patent- und Rechtsanwälte
(86) International application number: PCT/CN2019/097104
(87) International publication number: WO 2020/029788

(56) References cited:
- CN-A- 102 872 521
- CN-A- 104 645 480
- CN-A- 108 310 591
- CN-U- 209 451 112
- US-A1- 2007 142 785
- US-A1- 2009 043 260
- US-A1- 2011 319 830
- US-A1- 2015 246 207

## Description

### Technical Field

The invention relates to the technical field of medical instruments, in particular to a catheter positioning device for positioning a medical catheter at on a body.

### Background Art

In the medical field, a flexible catheter is required to be left in a human body to facilitate the input and output management of liquid under the conditions of thoracoabdominal postoperation, wound drainage, gastrointestinal nutrition transportation and so on, and the catheters usually need to be firmly fixed outside the human body.

At present, common catheter fixing methods include that one end of the catheter is attached and fixed on the skin of the human body by using an adhesive film product, namely a whole piece of general adhesive film, and the method is only suitable for fixing thin and soft indwelling catheters such as intravenous infusion catheters. In another method, a friction force is provided by applying pressure to the wall of the catheter along a radial direction so as to realize locking and fixing of the catheter; however, in the actual operation process, the situation that the strength is difficult to master exists, that is, when the radial pressure to the wall of the catheter is increased to improve the friction locking force, the inner diameter of the catheter is inevitably reduced due to compression, so that the efficiency of the catheter for draining liquid is decreased. If it is desired to minimize the effect on the drainage efficiency of the catheter, it is necessary to reduce the pressure on the wall of the catheter, but this results in a reduction of the frictional force on the wall of the catheter; and when the frictional force is reduced, the fixing effect on the catheter is greatly affected.

Due to the fact that the two types of catheter fixing modes are not ideal, more clinical medical workers choose a suture method to suture the catheter and the skin, which is painful for patients and has an occurrence rate at which a certain proportion of the catheters are torn off from the skin, and finally the catheter is displaced, so that the subsequent treatment of diseases is greatly affected.

US2007142785A1 discloses a device for securing a line such as a medical tube, a catheter, diagnostic wires etc., the device including a line retaining part and a base part, the line retaining part having two grooves for accommodating a line, the groove being designed with flexible retaining means in the form of flexible blades, the base part including fixing means, where the line retaining part and the base part are provided with complementary locking means.

In this prior art, the flexible blades are fixed to the retaining part, so once a tube is accommodated in the groove, the tube will be always gripped and pressed by the blades on the two opposite side of the groove, i.e. there is no a released state that liquid in the tube can flow without being intervened by the blades. Therefore, this device cannot be used to ensure the drainage of liquid through the tube when needed, and lock the tube to decrease the efficiency of draining liquid through the tube when not needed.

Therefore, how to provide the catheter positioning device so as to overcome various problems in the prior art is the technical problem to be solved in the case.

### Summary of the Invention

In view of the above problems, it is a primary object of the present application to provide a catheter positioning device having a self-triggering locking mechanism capable of achieving a one-way or two-way positioning locking function of the catheter to prevent displacement of the catheter.

The invention is set out in the appended set of claims.

According to the technical solution, the catheter positioning device provided by the embodiment of the invention has the advantages that the self-triggering type self-locking modules enable the self-locking modules to be in a released state without generating an obvious pressure on the outer pipe wall of the catheter when the catheter is positioned at the preset position in the positioning guide groove, so that the drainage efficiency of the catheter is not affected; and when the catheter is displaced in the positioning guide groove to deviate from the preset position, the self-locking modules are automatically switched from the released state to the locking state, so that the catheter is limited to move relative to the positioning guide groove, so as to achieve one-way or two-way catheter positioning effects. According to another embodiment of the self-locking modules, after the external force applied to the catheter is released, the self-locking modules are automatically recovered to the released state from the locking state, so that the drainage efficiency of the catheter is ensured.

### Brief Description of the Drawings

In order to illustrate the technical solution in this application embodiment or in the prior art more clearly, a brief description of the accompanying drawings required for use in the embodiment or the description of the prior art is given below. It is obvious that the accompanying drawings described below are only some of the embodiments recorded in this application. Other drawings will be acquired easily for those of ordinary skill in the art from the accompanying drawings.
Fig. 1A is a perspective view showing an overall structure of a catheter positioning device according to a first embodiment of the present application;
Figs. 1B and 1C are side and top views, respectively, of the catheter positioning device of Fig. 1A;
Fig. 1D is an exploded view showing the catheter positioning device of Fig. 1A;
Figs. 2A and 2B respectively show schematic views of internal structures at different viewing angles when a self-locking module of the catheter positioning device of Fig. 1A is in a released state;
Figs. 3A and 3B respectively show schematic views of internal structures at different viewing angles when the self-locking modules of the catheter positioning device of Fig. 1A are in a locking state;
Fig. 3C is a side view showing the self-locking modules shown in Figs. 3A and 3B;
Figs. 4A and 4B are schematic views showing different embodiments of a base of the catheter positioning device of the present application;
Fig. 5 is a view showing different embodiments of a positioning guide groove in the catheter positioning device of the present application;
Figs. 6A to 6D are views showing different embodiments of a self-locking unit and a contact portion in the catheter positioning device of the present application;
Figs. 7A to 7D are views showing different embodiments of the self-locking unit of the catheter positioning device of the present application;
Figs. 8A-B are views showing an embodiment of the self-locking modules of the catheter positioning device of the present application in a released state and a locking state;
Figs. 9A-B are views showing another embodiment of the self-locking modules of the catheter positioning device of the present application in a released state and a locking state;
Figs. 10A and 10B are views showing an embodiment of the self-locking modules of the catheter positioning device of the present application with a self-resetting effect;
Figs. 11A and 11B are views showing an embodiment of an auxiliary positioning unit in the catheter positioning device of the present application;
Figs. 12A and 12B are perspective views showing an overall structure of the catheter positioning device according to a second embodiment of the present invention; and
Figs. 13A-13E are views showing an embodiment of a position adjustment assembly in the catheter positioning device of the present application.

### Reference numerals of elements

10 Catheter positioning device
11 Base
110 Heightening unit
12 Positioning guide groove
121 Limit portion
122 Auxiliary positioning unit
13, 130a-130c Self-locking module
13ABody of self-locking module
13B Cover body of self-locking module
131 V-groove
1311 Top end
1312 Sidewall of first groove body
1313 Sidewall of second groove body
C1 Reference center surface of groove body
132 Self-locking unit
1321 Second terminal
1322, 1322a, 1322b Sidewall of self-locking unit
C2 Reference center surface of self-locking unit
133, 133a, 133b Contact portion (first terminal)
1330 Arc surface portion
1331 First end
1332 Second end
134 Substrate
14 Stop module
15 Position adjustment assembly
151 First position adjustment unit
152 First fixing unit
153 Second position adjustment unit
154 Second fixing unit
16 Adhesion unit
2 Catheter
21 Outer pipe wall

### Detailed Description of the Invention

In order to enable those skilled in the art to better understand the technical solutions in the embodiments of the present application, the technical solutions in the embodiments of the present application will be clearly and completely described below in conjunction with the drawings in the embodiments of the present application. Obviously, the described embodiments are only a part of the embodiments of the present application, rather than all the embodiments. Based on the embodiments of the present application, all other embodiments obtained by one of ordinary skill in the art should be within the scope of the embodiments of the present application.

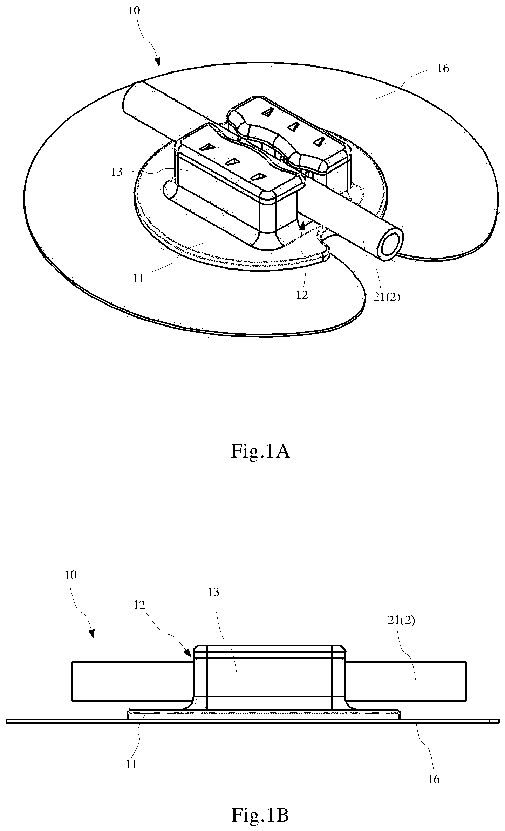

As shown in Fig. 1A, a catheter positioning device 10 of the application is configured for positioning a catheter 2 on a body (not shown). In this embodiment, the body is, for example, human skin, and the catheter 2 is, for example, a medical flexible catheter that remains in the body after thoracoabdominal surgery, wound drainage, gastrointestinal nutrition transportation, etc. The catheter positioning device 10 ensures the drainage effect of the catheter 2 by positioning an exposed part of the catheter 2 on the skin of the human body so that a built-in part of the catheter 2 remaining in the human body is not displaced.

Referring to Figs. 1A-1C in conjunction, the catheter positioning device 10 according to a first embodiment of the present invention generally includes a base 11, a positioning guide groove 12, and at least one self-locking module 13.

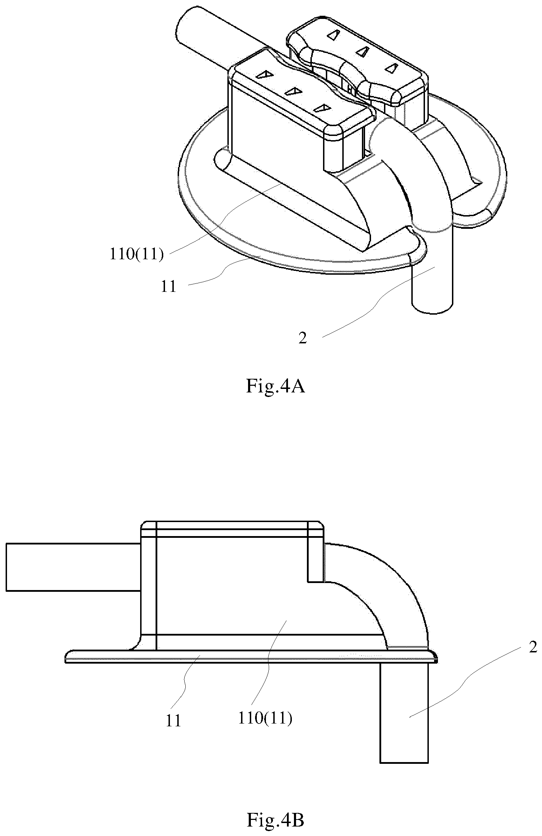

The base 11 is fixedly arranged on a body (not shown). In an embodiment, as shown in Figs. 1A-1C, the base 11 may be designed as a thin base, and when the base 11 is a thin base, a part of the catheter 2 may be arranged parallel to the surface of the body (see Fig. 1B). In another embodiment, as shown in Figs. 4A and 4B, the base 11 may also be designed as a thick base. When the base 11 is a thick base, a part of the catheter 2 may be arranged perpendicular to the surface of the body (see Fig. 4B). Although not limited thereto, the catheter 2 may be arranged on the surface of the body in an inclined manner by changing the inclination angle of the side surface of the catheter 2. In addition, according to the manufacturing method of the thick base 11, the thick base 11 can be integrally manufactured according to actual use requirements, and the manufacturing of the thick base can be indirectly realized by additionally arranging a heightening unit 110 on the basis of the thin base 11.

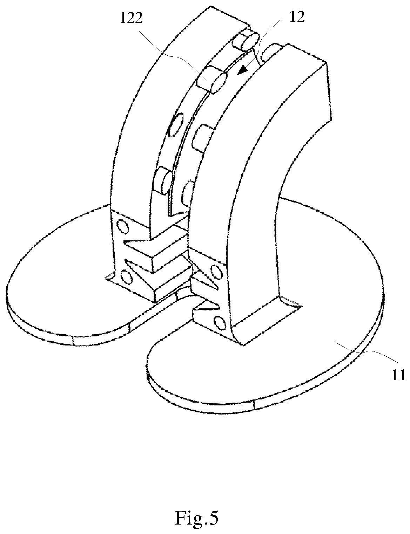

It should be noted that the arrangement between the catheter 2 and the surface of the body does not depend entirely on the thickness of the base 11. In yet another embodiment, as shown in Fig. 5, when the base 11 is a thin base, a part of the catheter 2 may be arranged perpendicular to the surface of the body by arranging the positioning guide groove 12 as a three-dimensional arc guide groove.

In addition, in this embodiment, the base 11 is adhesively fixed to the body (human skin); and the catheter positioning device 10 of the present application may further include an adhesion unit 16, as shown in Figs. 1A to 1C, wherein an upper surface of the adhesion unit 16 is fixed to a bottom of the base 11, and a lower surface of the adhesion unit 16 has an adhesive for adhering to the body, so that the base 11 is indirectly fixed to the body. However, without limitation thereto, other means of fixing the base 11 to the body may be used.

The positioning guide groove 12 is arranged on the base 11 for accommodating the catheter 2. In particular, the positioning guide groove 12 is configured for accommodating a part of the catheter 2 arranged on the surface of the body. In the present embodiment, the positioning guide groove 12 is designed as a planar structure as shown in Fig. 1A. In addition, the positioning guide groove 12 may be designed as a three-dimensional structure as shown in Fig. 5. However, it is not limited thereto, and in practical applications, the positioning guide groove 12 may be designed in other structural forms depending on practical requirements.

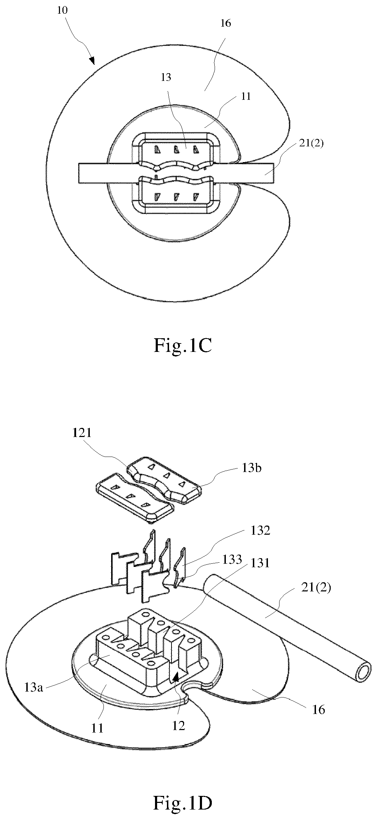

In addition, in order to make the catheter 2 not easily fall off from the positioning guide groove 12 during use, the positioning guide groove 12 of the present application also has a limit portion 121 for limiting the positioning of the catheter 2 in the positioning guide groove 12. As shown in Figs. 1A and 1C, in an embodiment, the limit portion 121 may be, for example, an undulating groove edge arranged at a notch of the positioning guide groove 12.

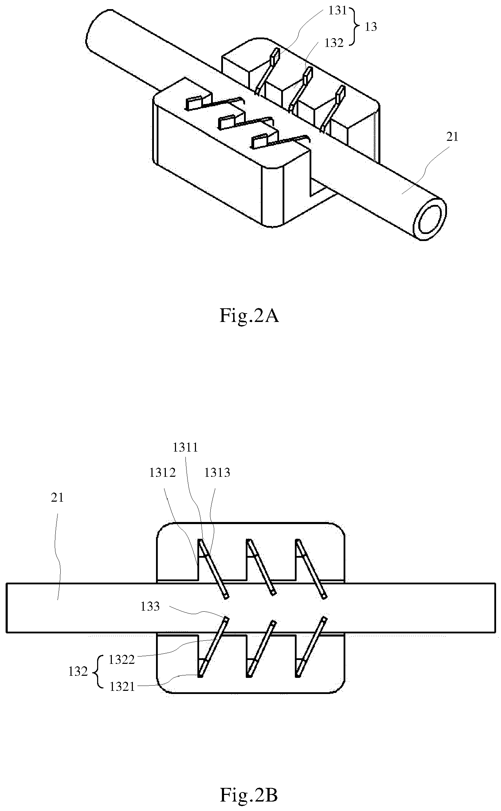

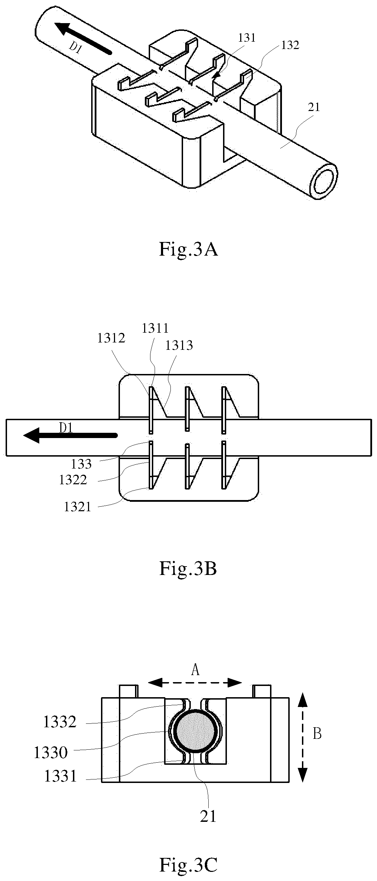

The self-locking modules 13 are arranged on the base 11. In this embodiment, the self-locking modules 13 may be positioned on opposite sides of the positioning guide groove 12, respectively. Specifically, in order to provide at least one self-locking module 13 on the opposite sides of the positioning guide groove 12, in the embodiment shown in Fig. 1A, three self-locking modules 13 are arranged on the opposite sides of the positioning guide groove 12, respectively. However, this is not a limitation. The specific number of arrangement of the self-locking modules 13 can be adjusted according to practical requirements. Furthermore, in a preferred embodiment, the self-locking modules 13 may, for example, be arranged in pairs on the opposite sides of the positioning guide groove 12 (as shown in Figs. 2A, 2B, 3A and 3B). Although not limited thereto, in other embodiments, the self-locking modules 13 may be arranged in a staggered manner on the opposite sides of the positioning guide groove 12.

In addition, the positioning guide groove 12 and the self-locking modules 13 can be designed in different structural forms according to different use requirements. Specifically, in the embodiment shown in Fig. 1A, the positioning guide groove 12 and the self-locking modules 13 are arranged on the upper surface of the thin base 11 (see Fig. 1A). In other embodiments, for example, when the base 11 is designed as a thick base, the positioning guide grooves 12 and the self-locking modules 13 may alternatively be arranged on the side surface of the base 11, in addition to being arranged on the upper surface of the base 11. In addition, when the base 11 has a three-dimensional arc guide groove structure as shown in Fig. 5, each self-locking module 13 may be arranged in a three-dimensional form along the arc three-dimensional structure of the positioning guide groove 12.

Referring to schematic views of internal structures of self-locking modules of Figs. 1D, 2A, 2B, 3A and 3B, a V-groove 131 and a locking unit 132 are formed in each locking module 13. The self-locking unit 132 is movably arranged in the V-groove 131, and a first terminal of the self-locking unit 132 is provided with a contact portion 133 which is in contact with an outer pipe wall 21 of the catheter 2 arranged in the positioning guide groove 12.

In an embodiment, the self-locking unit 132 may be made of, for example, a polymeric material or a metallic material, or a combination of the polymeric material and the metallic material. In a specific embodiment, the manufacturing material and the structural form of the self-locking unit 132 can be determined according to the material of the catheter 2 to be fixed. For example, if the catheter 2 is made of a relatively soft material (such as a hose made of silica gel, PU or TPU suitable for the abdominal cavity), the self-locking unit 132 can be made of a high-molecular material (such as plastic). If the catheter 2 is made of a relatively hard material (e.g. a rigid tube of PVC, PP, a modified PU material suitable for the thorax), the self-locking unit 132 may be made of a metal material.

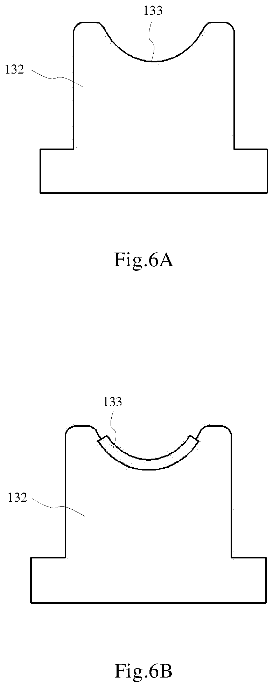

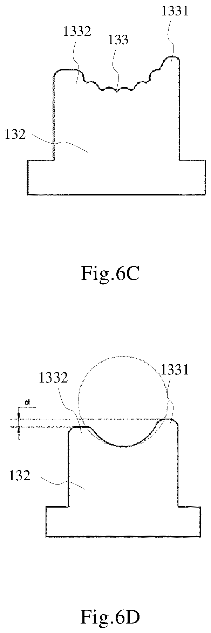

Referring to Figs. 6A-6D in conjunction, in an embodiment, the contact portion 133 may be integrally formed with the body of the self-locking unit 132 (as shown in Fig. 6A). In other embodiments, the contact portion 133 may be a separate part additionally provided on the self-locking unit 132, such as an adhesive tape (as shown in Fig. 6B). Furthermore, the shape of the contact portion 133 is adapted to the shape of the outer pipe wall 21 of the catheter 2, which in the present embodiment is, for example, a circular structure, and the contact portion 133 is accordingly designed as an arc-shaped structure (as shown in Figs. 2A to 2B, Figs. 3A to 3B, and Figs. 6A to 6D). In addition, the contact portion 133 may be designed to have a toothed structure or a toothless structure according to actual use requirements. Among them, when the self-locking unit 132 is made of a polymer material, it is suitably designed to have a toothless structure (as shown in Fig. 6A); and when the self-locking unit 132 is made of a metal material, it is preferably designed to have a toothed structure (as shown in Fig. 6C).

With continued reference to Figs. 6C and 6D, in another embodiment, the contact portion 133 further has a first end 1331 at the bottom of the positioning guide groove 12 and a second end 1332 at the notch of the positioning guide groove 12, wherein the second end 1332 may be slightly shorter in length than the first end 1331 to facilitate installation of the catheter 2 in the positioning guide groove 12.

Referring to Fig. 1D, in the actual manufacturing process, the positioning guide groove 12 and the bodies 13a of the plurality of self-locking modules 13 arranged on opposite sides of the positioning guide groove 12 can be designed to be integrally formed; the bodies 13a of the self-locking modules 13 are provided with a plurality of V-grooves 131 for arranging the self-locking units 132, and the self-locking modules 13 are also provided with cover bodies 13b which may cover the bodies 13a of the self-locking modules 13 to position the self-locking unit 132 within the V-groove 131 and to allow the self-locking unit 132 to be removably received within the V-groove 131. In the embodiment shown in Fig. 1D, the body 13a and the cover 13b of the self-locking modules 13 are separately designed, but not limited thereto. In other embodiments, the body 13a and the cover 13b may be pivotally designed. Further, in other embodiments, the above-mentioned limit portion 121 may be formed directly on one side edge of the cover body 13b of the self-locking modules 13 adjacent to the notch of the positioning guide groove 12.

With continued reference to Figs. 2A, 2B, 3A and 3B, the self-locking unit 132 of the present application can be switched between a released state (shown in Figs. 2A and 2B) and a locking state (shown in Figs. 3A to 3C) by being movable in the V-groove 131. Specifically, when the catheter 2 is positioned at a preset position within the positioning guide groove 12, each lock module 13 is in the released state, in which the catheter positioning device 10 hardly affects the pressure and the inner sectional area of the pipe wall of the pipe 2. Therefore, it does not affect the drainage efficiency of the catheter 2. When the catheter 2 moves relative to the positioning guide groove 12 to deviate from the preset position, the contact portion 133 of the self-locking unit 132 and the outer pipe wall 21 of the catheter 2 are in mutual contact, so that the self-locking unit 132 can be interlocked in the V-groove 131 together by a friction force generated by mutual contact between the contact portion 133 and the outer pipe wall 21; thereby, the released state is automatically switched to the locking state so as to restrict the movement of the catheter 2 relative to the positioning guide groove 12 by a mutual match between each locking module 13 in locking state arranged on the opposite sides of the positioning guide groove 12.

Specifically, as shown in Figs. 2B and 3B, the V-groove 131 of the present application is also provided with a top end 1311, a sidewall of a first groove body 1312 and a sidewall of a second groove body 1313 which extend from the top end 1311, and the sidewall of the first groove body 1312 and the sidewall of the second groove body 1313 are configured for defining the range of a movable section of the self-locking unit 132 in the V-groove 131.

The self-locking unit 132 has a second terminal 1321 opposite to the first terminal, and a sidewall 1322 of the self-locking unit extending from the second terminal 1321 to the first terminal (i.e., the position of the contact portion 133); and the contact portion 133 is arranged on the first terminal of the self-locking unit sidewall 1322. The second terminal 1321 of the self-locking unit 132 is positioned in the top end 1311 of the V-groove 131, and the length of the sidewall 1322 of the self-locking unit can be slightly longer than the depth of the V-groove 131, so that the contact portion 133 arranged on the first terminal slightly protrudes out of the V-groove 131 which is in contact with the outer pipe wall 21 of the catheter 2 arranged in the positioning guide groove 12. The self-locking unit 132 rotates reciprocatingly in the movable section of the V-groove 131 by taking the second terminal 1321 as a shaft end and is switched between the released state and the locking state.

When the self-locking unit 132 is in a released state, the contact portion 133 of the self-locking unit 132 contacts the outer pipe wall 21 of the catheter 2. In this state, the contact portion 133 hardly generates an acting force on the outer pipe wall 21 of the catheter 2. When the self-locking unit 132 is interlocked along with the displacement of the catheter 2 so as to switch from the released state to the locking state, the acting force generated by the contact portion 133 on the outer pipe wall 21 of the catheter 2 is gradually increased, so that the frictional force generated between the contact portion 133 and the outer pipe wall 21 is gradually increased, thereby generating sufficient frictional force between the contact portion 133 and the outer pipe wall 21 to restrict the outer pipe wall 21 from sliding relative to the contact portion 133, which thus indirectly achieves the technical effect of positioning the catheter 2 in the positioning guide groove 12.

The self-locking modules 13 of the present application may have a variety of configurations and arrangements thereof, and several representative embodiments will now be described by way of example. It should be noted that the embodiments set forth below are not intended to be exhaustive of all implementation forms of the self-locking modules 13, as previously described.

In an embodiment, the self-locking unit 132 of the present application is a sheet structure. In this case, the V-groove 131 mainly has the following design forms.

A first design form is shown in Figs. 2A to 2B and Figs. 3A to 3B. In this embodiment, the sidewall 1312 of the first groove body of the V-groove 131 is perpendicular to the axis of the catheter 2 arranged in the positioning guide groove 12. In this case, the self-locking unit 132 has a one-way self-locking function.

Specifically, as shown in Figs. 2A and 2B, when the catheter 2 is positioned at a preset position in the positioning guide groove 12, the self-locking unit sidewall 1322 of the self-locking unit 132 presents a state approaching to or abutting against the sidewall 1313 of the second groove body. At this time, the contact portion 133 of the self-locking unit 132 contacts the outer pipe wall 21 of the catheter 2, the contact portion 133 generates a minimal acting force to the outer pipe wall 21, and the self-locking unit 132 is in the released state.

As shown in Figs. 3A and 3B, when the catheter 2 moves to a first direction (for example, in a direction indicated by an arrow D1 in Fig. 3B) relative to the positioning guide groove 12 to deviate from the preset position, the self-locking unit sidewall 1322 of the self-locking unit 132 gradually moves from the sidewall 1313 of the second groove body of the V-groove 131 to the direction of the sidewall 1312 of the first groove body so as to approach to or directly abut against the sidewall 1312 of the first groove body. During the movement of the sidewall 1322 of the self-locking unit, the force generated by the contact portion 133 on the outer wall 21 of the catheter 2 is gradually increased, so that the self-locking unit 132 is gradually switched from the released state to the locking state. In this state, each locking unit 132, both in locking state, arranged on opposite sides of the positioning guide groove 12 will restrict the catheter 2 from continuing to move relative to the positioning guide groove 12 in the direction indicated by arrow D1, thereby achieving a one-way self-locking function.

Referring to Fig. 3C, as described above, in the present embodiment, the catheter 2 has a circular structure, and the contact portion 133 has an arc structure. In order to avoid the affection of the self-locking modules 13 in locking state on the drainage efficiency of the catheter 2, when the self-locking unit 132 of the present application is in locking state, the arc surface portions 1330 arranged in the arc-shaped structure of the contact portions 133 on opposite sides of the positioning guide groove 12 may be generated to the outer pipe wall 21 of the catheter 2 in a third direction (i.e., the direction of the axis A in Fig. 3C). At the same time, the first end 1331 and the second end 1332 of the contact portion 133 respectively generate a force on the outer pipe wall 21 of the catheter 2 in a fourth direction (i.e. the direction of the axis B in figure 3C) perpendicular to the third direction, so that the self-locking unit 132 generates a force on the outer pipe wall 21 of the catheter 2 in a surrounding manner when the self-locking unit 132 is in locking state. Therefore, the catheter 2 can still keep or basically keep the pipe wall of the catheter 2 in locking state to maintain the original shape and not easy to deform, so that the drainage efficiency of the catheter 2 is prevented from being affected.

In the other design form, the V-groove 131 also has a reference center surface of the groove body having an equal included angle with the sidewall of the first groove body 1312 and the sidewall of the second groove body 1313 respectively, and the reference center surface of the groove body is perpendicular to the axis of the catheter 2 arranged in the positioning guide groove 12. In this case, the self-locking unit 132 has a two-way self-locking function. Specifically, when the catheter 2 is positioned at a preset position within the positioning guide groove 12, the self-locking unit 132 is in the released state in which the self-locking unit sidewall 1322 of the self-locking unit 132 approaches to the sidewall 1312 of the first groove body or the sidewall 1313 of the second groove body or directly abuts against the sidewall 1312 of the first groove body or the sidewall 1313 of the second groove body. In this state, the contact portion 133 of the self-locking unit 131 contacts the outer pipe wall 21 of the catheter 2, and hardly generates an acting force on the outer pipe wall 21; when the catheter 2 moves to a first direction relative to the positioning guide groove 12 or a second direction opposite to the first direction relative to the positioning guide groove 12 to deviate from a preset position, the self-locking unit sidewall 1322 of the self-locking unit 132 is gradually rotated to the direction of the reference center surface of the groove body from the sidewall 1312 of the first groove body or the sidewall 1313 of the second groove body of the V-groove 131 so as to approach to the reference center surface of the groove body without completely coinciding with the reference center surface of the groove body. At this time, the acting force generated by the contact portion 133 on the outer pipe wall 21 of the pipe 2 is gradually increased, and the self-locking unit 132 is automatically switched from the released state to the locking state. In this state, the self-locking units 132 arranged on the opposite sides of the positioning guide groove 12 and both in locking state will restrict the movement of the catheter 2 relative to the positioning guide groove 12 in the first direction or the second direction opposite to the first direction, thereby realizing a two-way self-locking function. This embodiment has the advantage that the position of the self-locking unit 132 relative to the V-groove 131 (i.e. the self-locking unit 132 approaches to or abuts against the sidewall 1312 of the first groove body or the sidewall 1313 of the second groove body) can be adjusted only by adjusting the self-locking modules 13 in the released state without changing the design shape and position of the components in the catheter positioning device 10. That is, the self-locking moving direction of the self-locking unit 132 can be adjusted, and the method has the characteristic of high setting flexibility.

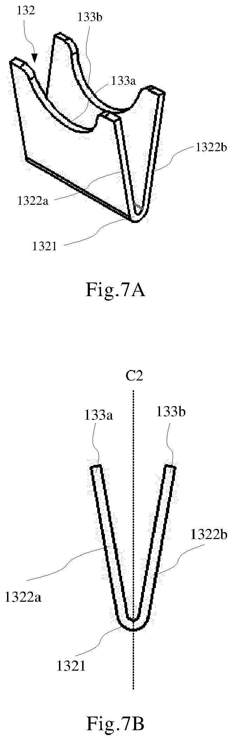

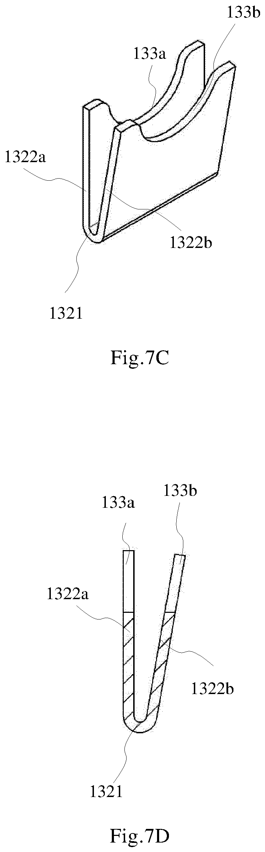

In another embodiment, the self-locking unit 132 of the present application is designed as a V-shaped sheet structure including a symmetrical V-shaped sheet structure as shown in Figs. 7A and 7B, or an asymmetrical V-shaped sheet structure as shown in Figs. 7C and 7D. In this case, the V-groove 131 mainly has the following design forms.

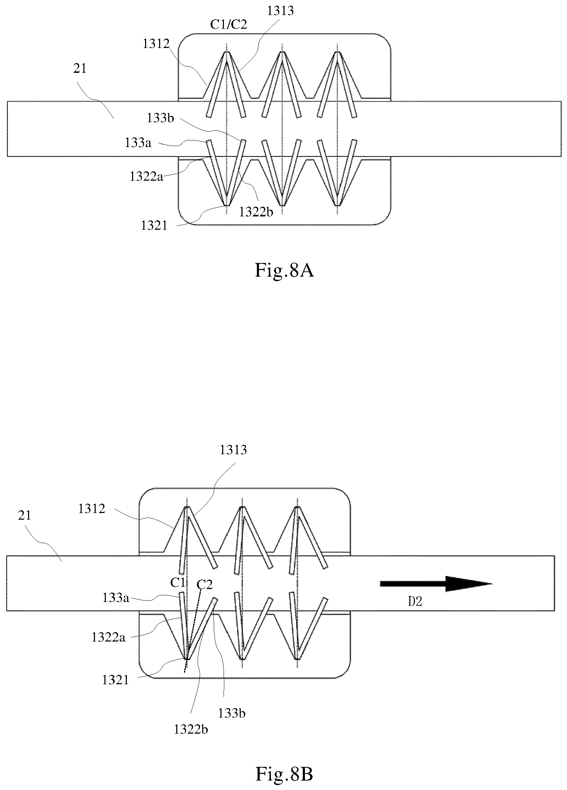

Referring now to Figs. 7A, 7B, 8A and 8B, in a first design form, a self-locking unit 132 (shown in Figs. 7A and 7B) having a symmetrical V-shaped sheet structure has one second terminal 1321, two first terminals, a sidewall 1322a of the first self-locking unit and a sidewall 1322b of the second self-locking unit respectively extending from the second terminal 1321 to the two first terminals, a first contact portion 133a and a second contact portion 133b respectively arranged on the two first terminals, and a reference center surface C2 of the self-locking unit has an equal included angle with the sidewall 1322a of the first self-locking unit and the sidewall 1322b of the second self-locking unit respectively. As shown in Figs. 8A and 8B, the V-groove 131 further has a reference center surface C1 having an equal included angle with the sidewall 1312 of the first groove body and the sidewall 1313 of the second groove body, respectively, wherein the reference center surface C1 of the groove body is perpendicular to the axis of the catheter 2 arranged in the positioning guide groove 12; and as seen from Figs. 8A and 8B, the opening angle of the self-locking unit 132 (i.e. the included angle formed between the sidewalls 1322a and 1322b of the self-locking unit) is slightly less than that of the V-groove 131 (i.e. the included angle formed between the sidewall 1312 of the first groove body and the sidewall 1313 of the second groove body), so that the self-locking unit 132 can move in the V-groove 131.

As shown in Fig. 8A, when the catheter 2 is positioned at a preset position in the positioning guide groove 12, the reference center surface C2 of the self-locking unit approaches to the reference center surface C1 of the groove body, or the reference center surface C2 of the self-locking unit and the reference center surface C1 of the groove body coincide with each other. At this time, the first contact portion 133a and the second contact portion 133b of the self-locking unit 132 simultaneously contact the outer pipe wall 21 of the catheter 2, and the self-locking unit 132 is in the released state.

In an embodiment, when the catheter 2 moves in a first direction relative to the positioning guide 12 (not shown), the self-locking unit 132 will be interlocked within the V-groove 131 with the movement of the catheter 2, so that the reference center surface C2 of the self-locking unit is offset from the reference center surface C1 of the groove body toward the direction of the sidewall 1312 of the first groove body, causing the first self-locking unit sidewall 1322a of the self-locking unit 132 to approach to or abut against the sidewall 1312 of the first groove body of the V-groove 131. At this time, the acting force generated by the second contact portion 133b on the outer pipe wall 21 of the catheter 2 is increased, and the self-locking unit 132 is switched from the released state to the locking state to limit the movement of the catheter 2 to the first direction relative to the positioning guide groove 12.

In another embodiment, as shown in Fig. 8B, when the catheter 2 moves to a second direction opposite to the first direction relative to the positioning guide 12 (e.g., the direction shown by arrow D2), the self-locking unit 132 will rotate in the V-groove 131 in the second direction (the direction shown by arrow D2 in Fig. 8B) along with the movement of the catheter 2, so that the reference center surface C2 of the self-locking unit is offset from the reference center surface C1 of the groove body to the direction of the sidewall 1313 of the second groove body. At this time, the second self-locking unit sidewall 1322b of the self-locking unit 132 gradually approaches to the V-groove 131 until it abuts against the sidewall 1313 of the second groove body of the V-groove 131; the force generated by the first contact portion 133a on the outer pipe wall 21 of the catheter 2 is increased, so that the self-locking unit 132 is automatically switched from a released state (the state shown in Fig. 8A) to a locking state (the state shown in Fig. 8B); therefore, the catheter 2 is restricted from moving in the second direction (the direction indicated by arrow D2 in Fig. 8B) relative to the positioning guide groove 12 by the sufficient friction generated between the first contact portion 133a and the outer pipe wall 21.

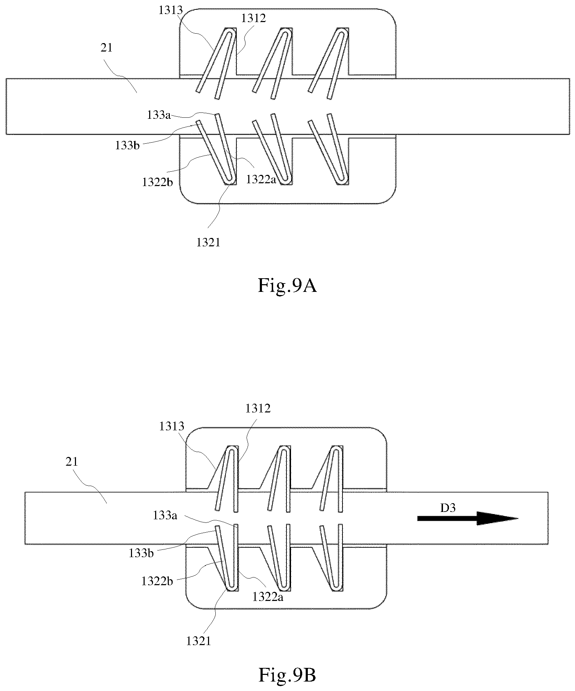

Referring to Figs. 7-C, 7-D, 9-A and 9-B, in another design form, the locking unit 132 (as shown in Figs. 7C and 7D) has one second terminal 1321, two first terminals, a sidewall 1322a of the first locking unit and a sidewall 1322b of the second locking unit extending from the second terminal 1321 to the two first terminals, respectively, and first and second contact portions 133a and 133b arranged on the two first terminals. The V-groove 131 also presents an asymmetric arrangement, i.e. the sidewall 1312 of the first groove body of the V-groove 131 is perpendicular to the axis of the catheter 2 arranged in the positioning guide groove 12. The opening angle of the self-locking unit 132 (i.e., the included angle formed between the sidewall 1322a of the first self-locking unit and the sidewall 1322b of the second self-locking unit) is also slightly less than that of the V-groove 131 (i.e., the included angle formed between the sidewall 1312 of the first groove body and the sidewall 1313 of the second groove body). In this embodiment, the self-locking unit 132 has a one-way self-locking function.

Specifically, as shown in Fig. 9A, when the catheter 2 is positioned at a preset position in the positioning guide groove 12, the second self-locking unit sidewall 1322b of the self-locking unit 132 approaches to or abuts against the sidewall 1313 of the second groove body of the V-groove 131. In this state, the first contact portion 133a contacts the outer pipe wall 21 of the catheter 2, and the self-locking unit 132 is in the released state.

As shown in Fig. 9B, when the catheter 2 moves to the first direction (for example, the direction indicated by arrow D3 in Fig. 9B) relative to the positioning guide groove 12 to deviate from the preset position, the self-locking unit 132 moves to the direction of the sidewall 1312 of the first groove body from the sidewall 1313 of the second groove body, so that the first self-locking unit sidewall 1322a of the self-locking unit 132 approaches to or abuts against the sidewall of the first groove body 1312. At this time, the first contact portion 133a on the first self-locking unit sidewall 1322a generates an increased force on the outer pipe wall 21 of the pipe 2, and the second contact portion 133b on the second self-locking unit sidewall 1322b also contacts the outer pipe wall 21 of the catheter 2 and the force applied to the outer pipe wall 21 of the catheter 2 are also gradually increased (in principle, the force generated by the second contact portion 133b to the outer pipe wall 21 of the catheter 2 is less than the force generated by the first contact portion 133a to the outer pipe wall 21 of the catheter 2). Simultaneously, the movement of the catheter 2 relative to the positioning guide groove 12 in the first direction (the direction shown by arrow D3 in Fig. 9B) are limited by the first contact portion 133a and the second contact portion 133b, so that the automatic switching process of the self-locking unit 132 from the released state shown in Fig. 9A to the locking state shown in Fig. 9B is completed, achieving the technical effect of self-triggering locking. An advantage of this embodiment is that a frictional restriction is generated for the movement of the catheter 2 by both the first contact portion 133a and the second contact portion 133b at the same time. Therefore, the positioning effect for the catheter 2 can be improved. In other embodiments of the present invention, the self-locking modules 13 may also have the technical effect of self-resetting to prevent the self-locking modules 13 in locking state from affecting the drainage efficiency of the catheter 2.

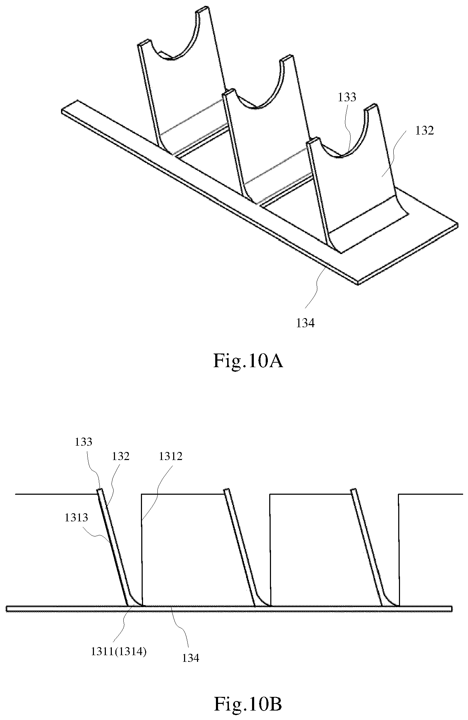

As shown in Figs. 10A and 10B, in the present embodiment, the self-locking modules 13 are further provided with a substrate 134, and the self-locking unit 132 is formed by bending and extending from the body of the substrate 134. The V-groove 131 is further provided with a top end 1311, a sidewall 1312 of the first groove body extending from the top end 1311, and a sidewall 1313 of the second groove body, wherein the sidewall 1312 of the first groove body of the V-groove 131 and the axis of the catheter 2 arranged in the positioning guide groove 12 are perpendicular to each other. In addition, a gap 1314 is formed at the top end 1311 of the V-groove 131, the substrate 134 is arranged on the base 11 and is positioned at the outer side of the V-groove 131, and the self-locking unit 132 bent and extended from the substrate 134 can pass through the gap 1314 positioned at the top end 1311 of the V-groove 131, extend from the outer side of the V-groove 131 to the inner side of the V-groove 131, and reciprocatingly in the movable section defined by the sidewall 1312 of the first groove body and the sidewall 1313 of the second groove body of the V-groove 131; and the contact portion 133 arranged on the first terminal of the self-locking unit 132 slightly protrudes out of the V-groove 131 and is in contact with the outer pipe wall 21 of the catheter 2 arranged in the positioning guide groove 12.

When the catheter 2 is positioned at a preset position in the positioning guide groove 12, the self-locking unit 132 presents a state that the sidewall 1313 of the second groove body of the V-groove 131 approaches to or abuts against the sidewall 1313 of the second groove body; at this time, the self-locking unit 132 is in a released state, and the substrate 134 is not deformed.

When the catheter 2 is subjected to an external force to move relative to the positioning guide groove 12 to deviate from the preset position and to be in the deviated position, the self-locking unit 132 rotates from the sidewall 1313 of the second groove body to the direction of the sidewall 1312 of the first groove body along with the moving direction of the catheter 2 by the contact portion 133 which is in contact with the outer pipe wall 21 of the catheter 2, so as to approach to the sidewall 1312 of the first groove body of the V-groove 131 or abut against the sidewall 1312 of the first groove body, so that the self-locking unit 132 is switched from the released state to the locking state, and the substrate 134 is deformed in locking state.

The deformed substrate 134 provides a self-resetting force to the self-locking unit 132 to cause the self-locking unit 132 to rotate from the first groove body sidewall 1312 to the second groove body sidewall 1313 so as to re-approach or abut against the second groove body sidewall 1313 when the external force applied to the catheter 2 is released and it recovers from the deviated position to the preset position, so that the self-locking modules 13 can automatically recover from the locking state to the released state under the action of the substrate 134. Since the contact portion 133 of the self-locking unit 132 in the released state hardly generates an acting force on the outer pipe wall 21 of the pipe 2, the affection on the liquid discharge efficiency of the pipe 2 can be avoided.

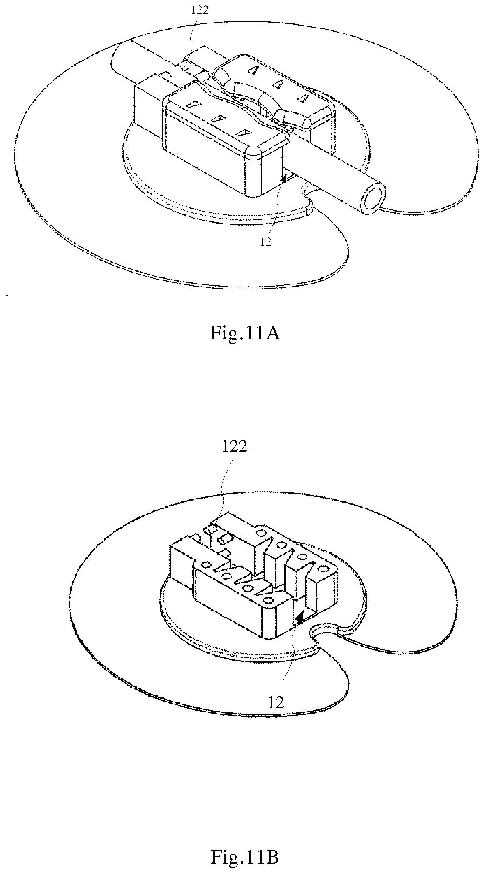

In other embodiments of the present application, in order to achieve a better positioning effect, a plurality of auxiliary positioning units 122 may be additionally provided in the positioning guide groove 12 of the present application for auxiliary positioning of the catheter 2 at a preset position in the positioning guide groove 12. As shown in Figs. 5, 11A and 11B, the auxiliary positioning unit 122 of the present application may be, for example, a plurality of auxiliary positioning bumps respectively arranged on the inner sidewall of the positioning guide groove 12.

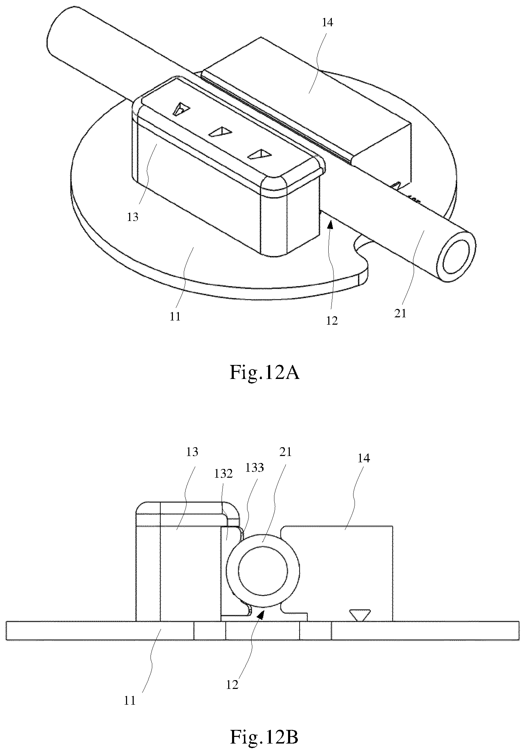

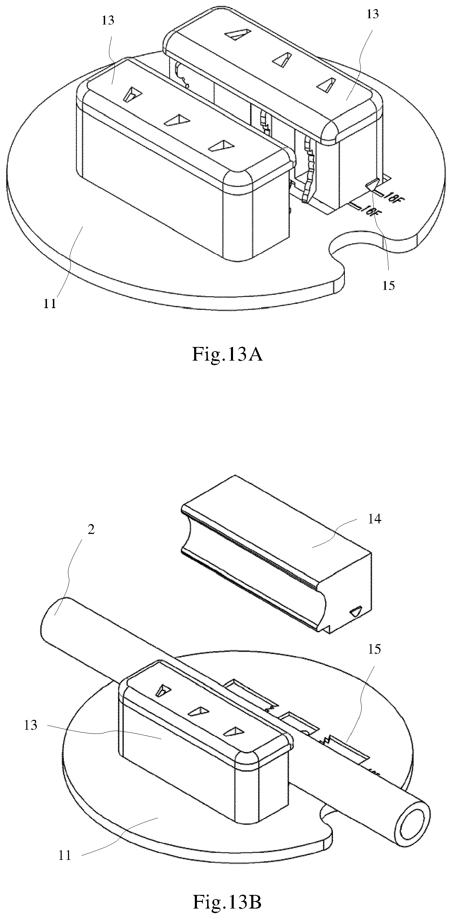

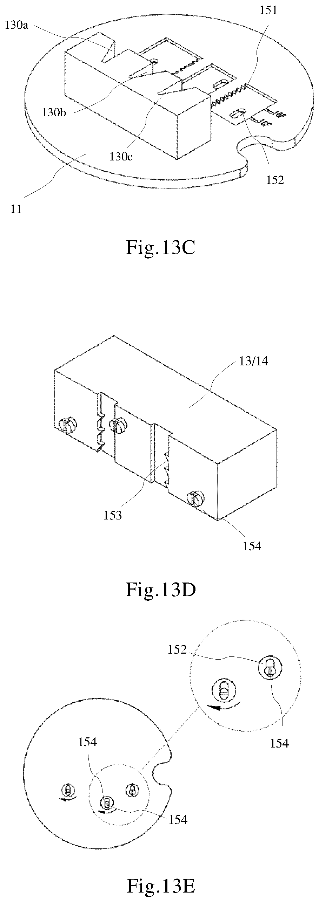

In another embodiment, the self-locking modules 13 may be arranged on only one side of the positioning guide groove 12, in addition to being arranged on opposite sides of the positioning guide groove 12. In particular, with reference to Figs. 12A and 12 B, in this embodiment, the catheter positioning device 10 further includes a stop module 14, wherein the stop module 14 is arranged on a base 11 at one side of the positioning guide groove 12, and the self-locking modules 13 are arranged on the base 11 at the other side of the positioning guide groove 12 relative to the stop module 14, wherein the self-locking modules 13 are also provided with a V-groove 131 and a self-locking unit 132, and the self-locking unit 132 is further provided with a contact portion 133 which can be matched with the stop module 14, so that the contact portion 133 is in contact with the outer pipe wall 21 of the catheter 2 arranged in the positioning guide groove 12. When the catheter 2 is positioned at the preset position of the positioning guide groove 12, the self-locking unit 132 is in the released state; when the catheter 2 moves relative to the positioning guide groove 12 to deviate from the preset position, the self-locking unit 132 is interlocked in the V-groove 131 by the contact portion 133 which is in contact with each other and the outer pipe wall 21 of the catheter 2 so as to automatically switch from the released state to the locking state; and the movement of the catheter 2 relative to the positioning guide 12 is limited by coordination of the self-locking unit 132 in locking state and the stop module 14. With continued reference to Figs. 13A to 13E, in order to achieve a positioning effect for catheters 2 of different pipe diameters, in another embodiment, the catheter positioning device 10 also has a position adjustment assembly 15 for providing an adjustment to the position at which the self-locking modules 13 (in the embodiment shown in Fig. 13A) or the stop module 14 (in the embodiment shown in Fig.l3B) are arranged on the base 11. In the present embodiment, the self-locking modules 13 or the stop module 14 arranged on one side of the positioning guide groove 11 can be set in the movable mode. by adjusting a specific position of the self-locking modules 13 or the stop module 14 in the movable mode arranged on the base 11, it achieves the technical effect of adjusting the distance between the self-locking modules 13 (or between the self-locking modules 13 and the stop modules 14) arranged on the opposite sides of the positioning guide groove 11, so as to adapt to the catheters 2 with different pipe diameters.

Specifically, the position adjustment assembly 15 includes a first position adjustment unit 151 and a first fixing unit 152 (shown in Fig. 13C) arranged on the base 11, and a second position adjustment unit 153 and a second fixing unit 154 arranged on a bottom surface of the self-locking modules 13 or the stop module 14 (i.e., a surface of the self-locking modules 13 or the stop module 14 contacting the base 11) (as shown in Fig. 13D), wherein the first position adjustment unit 151 and the second position adjustment unit 153 are matched mutually to adjust the position where the self-locking modules 13/the stop module 14 are mounted on the base 11, and the first fixing unit 152 and the second fixing unit 154 are matched mutually to fix the self-locking modules 13/the stop module 14 on the base 11. As shown in Figs. 13C to 13E, in the present embodiment, the first fixing unit 152 includes a plurality of oval-shaped openings formed in the base 11, and the second fixing unit 154 is a circular slider slidable with the first fixing unit 152, wherein the second fixing unit 154 is switchable between an unlocking state and a locking state by rotating with respect to the first fixing unit 152. When the second fixing unit is in a non-locking state, it can slide in the first fixing unit 152 to adjust the position of the self-locking modules 13/stop module 14 installed on the base 11. When the second fixing unit 154 is in locking state, the second fixing unit 154 can be locked at a desired position in the first fixing unit 152, and the self-locking modules 13/the stop module 14 can be fixed to the base 11. It should be noted that the above-described configuration of the first position adjustment unit 151, the first fixing unit 152, the second position adjustment unit 153, and the second fixing unit 154 is not limited to the embodiment shown in the drawings, and other configurations may be applicable to the present invention.

Moreover, in another embodiment, the limiting effect in different directions can be realized by different self-locking modules 13 arranged on the same base 11, and the technical effect of two-way self-locking can be realized for the catheter 2 of the positioning guide groove 12. Specifically, as shown in Fig. 13C, in the present embodiment, three self-locking modules 130a, 130b, 130c are arranged on the base 11, wherein the locking direction of the self-locking module 130a is opposite to the locking direction of the self-locking modules 130b and 130c, so that when the catheter 2 moves to the first direction relative to the positioning guide groove 12 to deviate from the preset position, the self-locking module 130a is automatically switched from a released state to a locking state so as to limit the movement of the catheter 2 relative to the positioning guide groove 12 in the first direction. On the contrary, when the catheter 2 moves to the second direction opposite to the first direction relative to the positioning guide groove 12 to deviate from the preset position, the self-locking modules 130b and 130c are automatically switched from the released state to the locking state so as to limit the catheter 2 to move relative to the positioning guide groove 12 in the second direction, achieving the technical effect of two-way locking.

According to the technical solution, by providing the catheter positioning device with a self-triggering locking mechanism, the one-way or two-way positioning function of the catheter can be realized. Under the condition that the catheter is not displaced, the affection of the catheter positioning device on the pressure and the inner sectional area of the wall of the catheter is minimal, so that the drainage efficiency of the catheter cannot be affected; and the self-locking unit in the catheter positioning device of the present invention can be instantaneously activated to switch from the released state to the locking state when the catheter is displaced or is about to be displaced by an external force, so that the frictional force between the catheter and the catheter positioning device is instantaneously increased to ensure that the catheter is firmly locked against displacement.

In addition, according to the catheter positioning device provided by another embodiment of the application, after the external force on the catheter is released, the self-locking unit in the catheter fixing device is automatically recovered to the released state from the locking state, so that the affection on the drainage efficiency of the catheter is avoided.

## Claims

1. A catheter positioning device (10) for positioning a catheter (2) on a body, comprising:
a base (11) fixedly arranged on the body;
a positioning guide groove (12) arranged on the base (11) and configured for accommodating the catheter (2); and
a plurality of self-locking modules (13, 130a-130c) arranged on the base (11) and positioned on two opposite sides of the positioning guide groove (12),
**characterized in that** each self-locking module (13, 130a-130c) comprises:
a V-groove (131); and
a self-locking unit (132) movably arranged in the V-groove (131), wherein a first terminal of the self-locking unit (132) is provided with a contact portion which is configured to be in contact with an outer pipe wall (21) of the catheter (2) arranged in the positioning guide groove (12), and the self-locking unit (132) moves in the V-groove (131) to switch between a released state and a locking state;
wherein, when the catheter (2) is positioned at a preset position in the positioning guide groove (12), each self-locking module (13, 130a-130c) is in the released state; when the catheter (2) moves relative to the positioning guide groove (12) to deviate from the preset position, the self-locking unit (132) is interlocked in the V-groove (131) by the contact portion and the outer pipe wall (21) of the catheter (2), and the self-locking unit (132) is automatically switched from the released state to the locking state, so that the movement of the catheter (2) relative to the positioning guide groove (12) is limited by mutual coordination of the self-locking modules (13, 130a-130c) which are arranged on the two opposite sides of the positioning guide groove (12) and are in locking state.

2. The catheter positioning device according to claim 1, **characterized in that** in the self-locking modules (13, 130a-130c),
the V-groove (131) is also provided with a top end (1311), a sidewall of a first groove body (1312) and a sidewall of a second groove body (1313) which extend from the top end (1311), and the sidewall of the first groove body (1312) and the sidewall of the second groove body (1313) are configured for defining a movable section in the V-groove (131);
the self-locking unit (132) further comprises a second terminal (1321) opposite to the first terminal, and a sidewall of the self-locking unit (1322, 1322a, 1322b) extending from the second terminal (1321) to the first terminal, wherein the second terminal (1321) of the self-locking unit (132) is positioned in the top end (1311) of the V-groove (131), and the length of the sidewall of the self-locking unit (1322, 1322a, 1322b) is slightly greater than the depth of the V-groove (131), so that the contact portion (133, 133a, 133b) arranged at the first terminal slightly protrudes out of the V-groove (131) so as to be in contact with the outer pipe wall (21) of the catheter (2) arranged in the positioning guide groove (12); and the self-locking unit (132) rotates reciprocatingly in the movable section of the V-groove (131) by taking the second terminal (1321) as a shaft end, and the self-locking unit (132) is switched between the released state and the locking state;
wherein, when the self-locking unit (132) is in the released state, the contact portion (133, 133a, 133b) of the self-locking unit (132) contacts the outer pipe wall (21) of the catheter (2); when the self-locking unit (132) is in locking state, an acting force generated by the contact portion (133, 133a, 133b) on the outer pipe wall (21) of the catheter (2) is increased, so that the frictional force generated between the contact portion (133, 133a, 133b) and the outer pipe wall (21) is increased, and the movement of the catheter (2) relative to the positioning guide groove (12) is limited by the sufficient frictional force generated between the contact portion (133, 133a, 133b) and the outer pipe wall (21).

3. The catheter positioning device according to claim 2, **characterized in that** the self-locking unit (132) is a sheet structure, the sidewall of the first groove body (1312) of the V-groove (131) is perpendicular to an axis of the catheter (2) arranged in the positioning guide groove (12), and the self-locking unit (132) has a one-way self-locking function;
wherein, when the catheter (2) is positioned at the preset position in the positioning guide groove (12), the sidewall of the self-locking unit (1322, 1322a, 1322b) approaches to the sidewall of the second groove body (1313) or abuts against the sidewall of the second groove body (1313), the contact portion (133, 133a, 133b) of the self-locking unit (132) contacts the outer pipe wall (21) of the catheter (2), and the self-locking unit (132) is in the released state;
when the catheter (2) moves to a first direction relative to the positioning guide groove (12) to deviate from the preset position, the sidewall of the self-locking unit (1322, 1322a, 1322b) rotates from the sidewall of the second groove body (1313) to the direction of the sidewall of the first groove body (1312) so as to approach to or abut against the sidewall of the first groove body (1312); and the acting force generated by the contact portion (133, 133a, 133b) on the outer pipe wall (21) of the catheter (2) is increased, and the self-locking unit (132) is switched from the released state to the locking state.

4. The catheter positioning device according to claim 2, **characterized in that** the self-locking unit (132) is a sheet structure, the V-groove (131) is further provided with a reference center surface of the groove body (C1) having an equal included angle with the sidewall of the first groove body (1312) and the sidewall of the second groove body (1313), the reference center surface of the groove body (C1) is perpendicular to an axis of the catheter (2) arranged in the positioning guide groove (12), and the self-locking unit (132) has a two-way self-locking function;
wherein, when the catheter (2) is positioned at the preset position in the positioning guide groove (12), the sidewall of the self-locking unit (1322, 1322a, 1322b) approaches to the sidewall of the first groove body (1312) or the sidewall of the second groove body (1313) or abuts against the sidewall of the first groove body (1312) or the sidewall of the second groove body (1313), the contact portion (133, 133a, 133b) of the self-locking unit (132) contacts the outer pipe wall (21) of the catheter (2), and the self-locking unit (132) is in the released state;
when the catheter (2) moves to a first direction relative to the positioning guide groove (12) or in a second direction opposite to the first direction relative to the positioning guide groove (12) to deviate from the preset position, the sidewall of the self-locking unit (1322, 1322a, 1322b) rotates from the sidewall of the first groove body (1312) or the sidewall of the second groove body (1313) to the direction of the reference center surface of the groove body (C1) so as to approach to the reference center surface of the groove body (C1); and the acting force generated by the contact portion (133, 133a, 133b) on the outer pipe wall (21) of the catheter (2) is increased, and the self-locking unit (132) is switched from the released state to the locking state.

5. The catheter positioning device according to claim 2, **characterized in that** the self-locking unit (132) is a V-shaped sheet structure, and has one second terminal (1321), two first terminals, a sidewall of the first self-locking unit (1322a) and a sidewall of the second self-locking unit (1322b) respectively extending from the one second terminal (1321) to the two first terminals, and a first contact portion (133a) and a second contact portion (133b) respectively arranged on the two first terminals, and a reference center surface of the self-locking unit (C2) having an equal included angle with the sidewall of the first self-locking unit (1322a) and the sidewall of the second self-locking unit (1322b) respectively; and the V-groove (131) is also provided with a reference center surface of the groove body (C1) having an equal included angle with the sidewall of the first groove body (1312) and the sidewall of the second groove body (1313) respectively, the reference center surface of groove body (C 1) is perpendicular to the axis of the catheter (2) arranged in the positioning guide groove (12), an opening angle of the self-locking unit (132) is slightly less than that of the V-groove (131), and the self-locking unit (132) has a two-way self-locking function;
wherein, when the catheter (2) is positioned at the preset position in the positioning guide groove (12), the reference center surface of the self-locking unit (C2) approaches to or coincides with the reference center surface of the groove body (C1), the first contact portion (133a) and the second contact portion (133b) of the self-locking unit (132) contact the outer pipe wall (21) of the catheter (2), and the self-locking unit (132) is in the released state;
when the catheter (2) moves to a first direction relative to the positioning guide groove (12) to deviate from the preset position, the reference center surface of the self-locking unit (C2) rotates from the reference center surface of the groove body (C1) to the direction of the sidewall of the first groove body (1312), so that the sidewall of the self-locking unit (1322, 1322a, 1322b) approaches or abuts against the sidewall of the first groove body (1312) of the V-groove (131); the acting force generated by the second contact portion (133b) on the outer pipe wall (21) of the catheter (2) is increased, and the self-locking unit (132) is switched from the released state to the locking state; or when the catheter (2) moves to a second direction opposite to the first direction relative to the positioning guide groove (12) to deviate from the preset position, the reference center surface of the self-locking unit (C2) rotates from the reference center surface of the groove body (C1) to the direction of the sidewall of the second groove body (1313), so that the sidewall of the second self-locking unit (1322b) approaches or abuts against the sidewall of the second groove body (1313) of the V-groove (131); and the acting force generated by the first contact portion (133a) on the outer pipe wall (21) of the catheter (2) is increased, and the self-locking unit (132) is switched from the released state to the locking state.

6. The catheter positioning device according to claim 2, **characterized in that** the self-locking unit (132) is a V-shaped sheet structure, and has one second terminal (1321), two first terminals, a sidewall of the first self-locking unit (1322a) and a sidewall of the second self-locking unit (1322b) respectively extending from the second terminal (1321) to the two first terminals, and a first contact portion (133a) and a second contact portion (133b) respectively arranged on the two first terminals; the sidewall of the first groove body (1312) of the V-groove (131) is perpendicular to an axis of the catheter (2) arranged in the positioning guide groove (12), the opening angle of the self-locking unit (132) is slightly less than that of the V-groove (131), and the self-locking unit (132) has a one-way self-locking function;
wherein, when the catheter (2) is positioned at the preset position in the positioning guide groove (12), the sidewall of the second self-locking unit (1322b) approaches or abuts against the sidewall of the second groove body (1313), the first contact portion (133a) contacts the outer pipe wall (21) of the catheter (2), and the self-locking unit (132) is in the released state;
when the catheter (2) moves to a first direction relative to the positioning guide groove (12) to deviate from the preset position, the self-locking unit (132) rotates from the sidewall of the second groove body (1313) to the direction of sidewall of the first groove body (1312), so that the sidewall of the first self-locking unit (1322a) approaches or abuts against the sidewall of the first groove body (1312), and the acting force generated by the first contact portion (133a) on the outer pipe wall (21) of the catheter (2) is increased; meanwhile, the second contact portion (133b) contacts the outer pipe wall (21) of the catheter (2) and increases the acting force generated on the outer pipe wall (21) of the catheter (2), so that the movement of the catheter (2) relative to the positioning guide groove (12) is limited by the first contact portion (133a) and the second contact portion (133b), and the self-locking unit (132) is switched from the released state to the locking state.

7. The catheter positioning device according to claim 1, **characterized in that** the self-locking modules (13, 130a-130c) further comprises a substrate (134), and the self-locking unit (132) is formed by bending and extending from the body of the substrate (134);
wherein the V-groove (131) is further provided with a top end (1311), a sidewall of the first groove body (1312) and a sidewall of the second groove body (1313) which extend from the top end (1311), and the top end (1311) is further provided with a gap, and the sidewall of the first groove body (1312) is perpendicular to the axis of the catheter (2) arranged in the positioning guide groove (12);
the substrate (134) is arranged on the base (11) and positioned on an outer side of the V-groove (131), the self-locking unit (132) bent and extended from the substrate (134) can pass through the gap to extend from the outer side of the V-groove (131) to an inner part of the V-groove (131), and the self-locking unit (132) rotates reciprocatingly in a movable section defined by the sidewall of the first groove body (1312) and the sidewall of the second groove body (1313); and the contact portion (133, 133a, 133b) arranged on the first terminal of the self-locking unit (132) slightly protrudes out of the V-groove (131) and is in contact with the outer pipe wall (21) of the catheter (2) arranged in the positioning guide groove (12);
wherein, when the catheter (2) is positioned at the preset position in the positioning guide groove (12), the self-locking unit (132) approaches to the sidewall of the second groove body (1313) or abuts against the sidewall of the second groove body (1313) to be in the released state, and the substrate (134) is not deformed; when the catheter (2) moves relative to the positioning guide groove (12) to deviate from the preset position to be in the deviated position, the self-locking unit (132) rotates from the sidewall of the second groove body (1313) to the direction of the sidewall of the first groove body (1312) so as to approach to the sidewall of the first groove body (1312) or abut against the sidewall of the first groove body (1312), so that the self-locking unit (132) is switched from the released state to the locking state, and the substrate (134) is deformed;
wherein, when the catheter (2) is recovered to be positioned at the preset position from the deviated position, the deformed substrate (134) provides a self-resetting acting force for the self-locking unit (132), so that the self-locking unit (132) rotates from the sidewall of the first groove body (1312) to the direction of the sidewall of the second groove body (1313) so as to approach to or abut against the sidewall of the second groove body (1313), and the self-locking modules (13, 130a-130c) are automatically recovered to the released state.

8. The catheter positioning device according to claim 1, **characterized in that** the shape of the contact portion (133, 133a, 133b) is adapted to the shape of the outer pipe wall (21) of the catheter (2), the catheter (2) is a circular structure, the contact portion (133, 133a, 133b) is an arc-shaped structure, and the contact portion (133, 133a, 133b) further has a first end (1331) positioned at a groove bottom of the positioning guide groove (12) and a second end (1332) positioned at a notch of the positioning guide groove (12), wherein the second end (1332) is slightly shorter in length than the first end (1331) to mount the catheter (2) in the positioning guide groove (12).

9. The catheter positioning device according to claim 8, **characterized in that** when the self-locking unit (132) is in locking state, the arc surface portion (1330) in the arc-shaped structure of the contact portion (133, 133a, 133b) generates an acting force on the outer pipe wall (21) of the catheter (2) in a third direction; and simultaneously, the first end (1331) and the second end (1332) of the contact portion (133, 133a, 133b) respectively generate an acting force on the outer pipe wall (21) of the catheter (2) in a fourth direction perpendicular to the third direction.

10. The catheter positioning device according to claim 1, **characterized in that** the shape of the contact portion (133, 133a, 133b) is adapted to the shape of the outer pipe wall (21) of the catheter (2), the self-locking modules (13, 130a-130c) are arranged in pairs or in a staggered manner on opposite sides of the positioning guide groove (12).

11. The catheter positioning device according to claim 1, **characterized in that** the positioning guide groove (12) further has a limit portion (121) for restricting the positioning of the catheter (2) in the positioning guide groove (12), the limit portion (121) is an undulating groove edge provided at a notch of the positioning guide groove (12).

12. The catheter positioning device according to claim 1, **characterized in that** a plurality of auxiliary positioning units (122) are further provided in the positioning guide groove (12) for auxiliary positioning of the catheter (2) at the preset position in the positioning guide groove (12).

13. The catheter positioning device according to claim 1, **characterized in that** the catheter positioning device (10) further comprises a position adjustment assembly for providing an adjustment to a position at which the self-locking modules (13, 130a-130c) are arranged on the base (11).

14. The catheter positioning device according to claim 1, **characterized in that**
when the catheter (2) moves to a first direction relative to the positioning guide groove (12) to deviate from the preset position, a part of the self-locking modules (13, 130a-130c) are automatically switched to the locking state from the released state so as to limit the catheter (2) to move relative to the positioning guide groove (12) in the first direction; and
when the catheter (2) moves relative to the positioning guide groove (12) in a second direction opposite to the first direction to deviate from the preset position, the other part of the self-locking modules (13, 130a-130c) are automatically switched from the released state to the locking state so as to limit the catheter (2) to move relative to the positioning guide groove (12) in the second direction.

## Patentansprüche

1. Katheter-Positionierung-Vorrichtung (10) zum Positionieren eines Katheters (2) an einem Körper, aufweisend:
eine Basis (11), welche fest an dem Körper angeordnet ist,
eine Positionierungsführungsnut (12), welche an der Basis (11) angeordnet und zum Beherbergen des Katheters (2) konfiguriert ist, und
mehrere Selbstarretierung-Module (13, 130a-130c), welche an der Basis (11) angeordnet und an zwei gegenüberliegenden Seiten der Positionierungsführungsnut (12) positioniert sind,
**dadurch gekennzeichnet, dass** jedes Selbstarretierung-Modul (13, 130a-130c) aufweist:
eine V-Nut (131), und
eine Selbstarretierung-Einheit (132), welche bewegbar in der V-Nut (131) angeordnet ist, wobei ein erster Endabschnitt der Selbstarretierung-Einheit (132) mit einem Kontaktabschnitt versehen ist, welcher konfiguriert ist, um mit einer äußeren Schlauchwand (21) des Katheters (2), welcher in der Positionierungsführungsnut (12) angeordnet ist, in Kontakt zu stehen, und wobei sich die Selbstarretierung-Einheit (132) in der V-Nut (131) bewegt, um zwischen einem Freigabe-Zustand und einem Arretierung-Zustand zu schalten,
wobei, wenn der Katheter (2) an einer vordefinierten Position in der Positionierungsführungsnut (12) positioniert ist, sich jedes Selbstarretierung-Modul (13, 130a-130c) in dem Freigabe-Zustand befindet, wobei, wenn sich der Katheter (2) relativ zu der Positionierungsführungsnut (12) bewegt, um von der vordefinierten Position abzuweichen, die Selbstarretierung-Einheit (132) in der V-Nut (131) ineinandergreifend arretiert ist mittels des Kontaktabschnitts und der äußeren Schlauchwand (21) des Katheters (2), und wobei die Selbstarretierung-Einheit (132) automatisch von dem Freigabe-Zustand aus zu dem Arretierung-Zustand geschaltet wird, so dass die Bewegung des Katheters (2) relativ zu der Positionierungsführungsnut (12) begrenzt wird mittels wechselseitigen Zusammenspiels der Selbstarretierung-Module (13, 130a-130c), welche an den zwei gegenüberliegenden Seiten der Positionierungsführungsnut (12) angeordnet sind und sich in einem Arretierung-Zustand befinden.

2. Katheter-Positionierung-Vorrichtung gemäß Anspruch 1, **dadurch gekennzeichnet, dass** in den Selbstarretierung-Modulen (13, 130a-130c) die V-Nut (131) auch mit einem oberen Ende (1311), einer Seitenwand eines ersten Nutkörpers (1312) und einer Seitenwand eines zweiten Nutkörpers (1313) versehen ist, welche sich von dem oberen Ende (1311) aus erstrecken, und wobei die Seitenwand des ersten Nutkörpers (1312) und die Seitenwand des zweiten Nutkörpers (1313) konfiguriert sind zum Definieren eines Bewegung-Abschnitts in der V-Nut (131),
wobei die Selbstarretierung-Einheit (132) ferner aufweist: einen zweiten Endabschnitt (1321), welcher entgegengesetzt zu dem ersten End-Abschnitt ist, und eine Seitenwand der Selbstarretierung-Einheit (1322, 1322a, 1322b), welche sich von dem zweiten Endabschnitt (1321) aus zu dem ersten Endabschnitt hin erstreckt, wobei der zweite Endabschnitt (1321) der Selbstarretierung-Einheit (132) in dem oberen Ende (1311) der V-Nut (131) angeordnet ist, und die Länge der Seitenwand der Selbstarretierung-Einheit (1322, 1322a, 1322b) geringfügig größer ist als die Tiefe der V-Nut (131), so dass der Kontaktabschnitt (133, 133a, 133b), welcher an dem ersten Endabschnitt angeordnet ist, geringfügig aus der V-Nut (131) herausragt, um in Kontakt mit der äußeren Schlauchwand (21) des Katheters (2) zu stehen, welcher in der Positionierungsführungsnut (12) angeordnet ist, und wobei die Selbstarretierung-Einheit (132) sich in dem Bewegung-Abschnitt der V-Nut (131) hin und her dreht mittels Verwendens des zweiten Endabschnitts (1321) als ein Achsenende, und wobei die Selbstarretierung-Einheit (132) zwischen dem Freigabe-Zustand und dem Arretierung-Zustand geschaltet wird,
wobei, wenn sich die Selbstarretierung-Einheit (132) in dem Freigabe-Zustand befindet, der Kontaktabschnitt (133, 133a, 133b) der Selbstarretierung-Einheit (132) die äußere Schlauchwand (21) des Katheters (2) berührt, wobei, wenn sich die Selbstarretierung-Einheit (132) in dem Arretierung-Zustand befindet, eine Wirkkraft, welche mittels des Kontaktabschnitts (133, 133a, 133b) auf die äußere Schlauchwand (21) des Katheters (2) erzeugt wird, erhöht wird, so dass die Reibungskraft, welche zwischen dem Kontaktabschnitt (133, 133a, 133b) und der äußeren Schlauchwand (21) erzeugt wird, erhöht wird, und die Bewegung des Katheters (2) relativ zu der Positionierungsführungsnut (12) begrenzt wird mittels der ausreichenden Reibungskraft, welche zwischen dem Kontaktabschnitt (133, 133a, 133b) und der äußeren Schlauchwand (21) erzeugt wird.

3. Katheter-Positionierung-Vorrichtung gemäß Anspruch 2, **dadurch gekennzeichnet, dass** die Selbstarretierung-Einheit (132) eine Blättchenstruktur ist, die Seitenwand des ersten Nutkörpers (1312) der V-Nut (131) senkrecht zu einer Achse des Katheters (2) ist, welcher in der Positionierungsführungsnut (12) angeordnet ist, und die Selbstarretierung-Einheit (132) eine Ein-Weg-Selbstarretierung-Funktion hat,
wobei, wenn der Katheter (2) an der vordefinierten Position in der Positionierungsführungsnut (12) positioniert ist, sich die Seitenwand der Selbstarretierung-Einheit (1322, 1322a, 1322b) der Seitenwand des zweiten Nutkörpers (1313) nähert oder an der Seitenwand des zweiten Nutkörpers (1313) anliegt, wobei der Kontaktabschnitt (133, 133a, 133b) der Selbstarretierung-Einheit (132) die äußere Schlauchwand (21) des Katheters (2) berührt, und wobei sich die Selbstarretierung-Einheit (132) in dem Freigabe-Zustand befindet,
wobei, wenn sich der Katheter (2) in einer ersten Richtung relativ zu der Positionierungsführungsnut (12) bewegt, um von der vordefinierten Position abzuweichen, sich die Seitenwand der Selbstarretierung-Einheit (1322, 1322a, 1322b) von der Seitenwand des zweiten Nutkörpers (1313) aus hin zu der Richtung der Seitenwand des ersten Nutkörpers (1312) dreht, um sich der Seitenwand des ersten Nutkörpers (1312) zu nähern oder daran anzuliegen, und wobei die Wirkkraft, welche mittels des Kontaktabschnitts (133, 133a, 133b) auf die äußere Schlauchwand (21) des Katheters (2) erzeugt wird, erhöht wird, und wobei die Selbstarretierung-Einheit (132) von dem Freigabe-Zustand aus zu dem Arretierung-Zustand geschaltet wird.

4. Katheter-Positionierung-Vorrichtung gemäß Anspruch 2, **dadurch gekennzeichnet, dass** die Selbstarretierung-Einheit (132) eine Blättchenstruktur ist, die V-Nut (131) ferner mit einer Referenz-Mittelfläche des Nutkörpers (C1) versehen ist, welche einen gleichen eingeschlossenen Winkel mit der Seitenwand des ersten Nutkörpers (1312) und der Seitenwand des zweiten Nutkörpers (1313) hat, wobei die Referenz-Mittelfläche des Nutkörpers (C1) senkrecht zu einer Achse des Katheters (2) ist, welcher in der Positionierungsführungsnut (12) angeordnet ist, und wobei die Selbstarretierung-Einheit (132) eine Zwei-Wege-Selbstarretierung-Funktion hat,
wobei, wenn der Katheter (2) an der vordefinierten Position in der Positionierungsführungsnut (12) positioniert ist, sich die Seitenwand der Selbstarretierung-Einheit (1322, 1322a, 1322b) der Seitenwand des ersten Nutkörpers (1312) oder der Seitenwand des zweiten Nutkörpers (1313) nähert oder an der Seitenwand des ersten Nutkörpers (1312) oder der Seitenwand des zweiten Nutkörpers (1313) anliegt, wobei der Kontaktabschnitt (133, 133a, 133b) der Selbstarretierung-Einheit (132) die äußere Schlauchwand (21) des Katheters (2) berührt, und wobei sich die Selbstarretierung-Einheit (132) in dem Freigabe-Zustand befindet,
wobei, wenn sich der Katheter (2) zu einer ersten Richtung relativ zu der Positionierungsführungsnut (12) hinbewegt oder sich in einer zweiten Richtung, welche entgegengesetzt zu der ersten Richtung ist, relativ zu der Positionierungsführungsnut (12) bewegt, um von der vordefinierten Position abzuweichen, sich die Seitenwand der Selbstarretierung-Einheit (1322, 1322a, 1322b) von der Seitenwand des ersten Nutkörpers (1312) oder der Seitenwand des zweiten Nutkörpers (1313) aus hin zu der Richtung der Referenz-Mittelfläche des Nutkörpers (C1) dreht, um sich der Referenz-Mittelfläche des Nutkörpers (C1) zu nähern, und wobei die Wirkkraft, welche mittels des Kontaktabschnitts (133, 133a, 133b) auf die äußere Schlauchwand (21) des Katheters (2) erzeugt wird, erhöht wird, und wobei die Selbstarretierung-Einheit (132) von dem Freigabe-Zustand aus zu dem Arretierung-Zustand geschaltet wird.

5. Katheter-Positionierung-Vorrichtung gemäß Anspruch 2, **dadurch gekennzeichnet, dass** die Selbstarretierung-Einheit (132) eine V-förmige Blättchenstruktur ist und hat: einen zweiten Endabschnitt (1321), zwei erste Endabschnitte, eine Seitenwand der ersten Selbstarretierung-Einheit (1322a) und eine Seitenwand der zweiten Selbstarretierung-Einheit (1322b), welche sich in jeweils zugeordneter Weise von dem einen zweiten Endabschnitt (1321) aus hin zu den zwei ersten Endabschnitten erstrecken, und einen ersten Kontaktabschnitt (133a) und einen zweiten Kontaktabschnitt (133b), welche in jeweils zugeordneter Weise an den zwei ersten Endabschnitten angeordnet sind, und eine Referenz-Mittelfläche der Selbstarretierung-Einheit (C2), welche in jeweils zugeordneter Weise einen gleichen eingeschlossenen Winkel mit der Seitenwand der ersten Selbstarretierung-Einheit (1322a) und der Seitenwand der zweiten Selbstarretierung-Einheit (1322b) hat; und wobei die V-Nut (131) auch mit einer Referenz-Mittelfläche des Nutkörpers (C1) versehen ist, welche in jeweils zugeordneter Weise einen gleichen eingeschlossenen Winkel mit der Seitenwand des ersten Nutkörpers (1312) und der Seitenwand des zweiten Nutkörpers (1313) hat, wobei die Referenz-Mittelfläche des Nutkörpers (C1) senkrecht zu der Achse des Katheters (2) ist, welcher in der Positionierungsführungsnut (12) angeordnet ist, wobei ein Öffnungswinkel der Selbstarretierung-Einheit (132) geringfügig kleiner ist als jener der V-Nut (131), und wobei die Selbstarretierung-Einheit (132) eine Zwei-Wege-Selbstarretierung-Funktion hat,
wobei, wenn der Katheter (2) an der vordefinierten Position in der Positionierungsführungsnut (12) positioniert ist, sich die Referenz-Mittelfläche der Selbstarretierung-Einheit (C2) der Referenz-Mittelfläche des Nutkörpers (C1) nähert oder mit jener übereinstimmt, wobei der erste Kontaktabschnitt (133a) und der zweite Kontaktabschnitt (133b) der Selbstarretierung-Einheit (132) die äußere Schlauchwand (21) des Katheters (2) berühren, und wobei sich die Selbstarretierung-Einheit (132) in dem Freigabe-Zustand befindet,
wobei, wenn sich der Katheter (2) zu einer ersten Richtung relativ zu der Positionierungsführungsnut (12) hinbewegt, um von der vordefinierten Position abzuweichen, sich die Referenz-Mittelfläche der Selbstarretierung-Einheit (C2) von der Referenz-Mittelfläche des Nutkörpers (C1) aus hin zu der Richtung der Seitenwand des ersten Nutkörpers (1312) dreht, so dass sich die Seitenwand der Selbstarretierung-Einheit (1322, 1322a, 1322b) der Seitenwand des ersten Nutkörpers (1312) der V-Nut (131) nähert oder an jener anliegt, wobei die Wirkkraft, welche mittels des zweiten Kontaktabschnitts (133b) auf die äußere Schlauchwand (21) des Katheters (2) erzeugt wird, erhöht wird, und wobei die Selbstarretierung-Einheit (132) von dem Freigabe-Zustand aus zu dem Arretierung-Zustand geschaltet wird; oder, wenn sich der Katheter (2) zu einer zweiten Richtung, welche entgegengesetzt zu der ersten Richtung ist, relativ zu der Positionierungsführungsnut (12) hinbewegt, um von der vordefinierten Position abzuweichen, sich die Referenz-Mittelfläche der Selbstarretierung-Einheit (C2) von der Referenz-Mittelfläche des Nutkörpers (C1) aus hin zu der Richtung der Seitenwand des zweiten Nutkörpers (1313) dreht, so dass sich die Seitenwand der zweiten Selbstarretierung-Einheit (1322b) der Seitenwand des zweiten Nutkörpers (1313) der V-Nut (131) nähert oder daran anliegt, und wobei die Wirkkraft, welche mittels des ersten Kontaktabschnitts (133a) auf die äußere Schlauchwand (21) des Katheters (2) erzeugt wird, erhöht wird, und wobei die Selbstarretierung-Einheit (132) von dem Freigabe-Zustand aus zu dem Arretierung-Zustand geschaltet wird.

6. Katheter-Positionierung-Vorrichtung gemäß Anspruch 2, **dadurch gekennzeichnet, dass** die Selbstarretierung-Einheit (132) eine V-förmige Blättchenstruktur ist und hat: einen zweiten Endabschnitt (1321), zwei erste Endabschnitte, eine Seitenwand der ersten Selbstarretierung-Einheit (1322a) und eine Seitenwand der zweiten Selbstarretierung-Einheit (1322b), welche sich in jeweils zugeordneter Weise von dem zweiten Endabschnitt (1321) aus hin zu den zwei ersten Endabschnitten erstrecken, und einen ersten Kontaktabschnitt (133a) und einen zweiten Kontaktabschnitt (133b), welche in jeweils zugeordneter Weise an den zwei ersten Endabschnitten angeordnet sind; wobei die Seitenwand des ersten Nutkörpers (1312) der V-Nut (131) senkrecht zu einer Achse des Katheters (2) ist, welcher in der Positionierungsführungsnut (12) angeordnet ist, wobei der Öffnungswinkel der Selbstarretierung-Einheit (132) geringfügig kleiner als jener der V-Nut (131) ist, und wobei die Selbstarretierung-Einheit (132) eine Ein-Weg-Selbstarretierung-Funktion hat,
wobei, wenn der Katheter (2) an der vordefinierten Position in der Positionierungsführungsnut (12) positioniert ist, sich die Seitenwand der zweiten Selbstarretierung-Einheit (1322b) der Seitenwand des zweiten Nutkörpers (1313) nähert oder daran anliegt, wobei der erste Kontaktabschnitt (133a) die äußere Schlauchwand (21) des Katheters (2) berührt, und wobei sich die Selbstarretierung-Einheit (132) in dem Freigabe-Zustand befindet,
wobei, wenn sich der Katheter (2) hin zu einer ersten Richtung relativ zu der Positionierungsführungsnut (12) bewegt, um von der vordefinierten Position abzuweichen, sich die Selbstarretierung-Einheit (132) von der Seitenwand des zweiten Nutkörpers (1313) aus hin zu der Richtung der Seitenwand des ersten Nutkörpers (1312) dreht, so dass sich die Seitenwand der ersten Selbstarretierung-Einheit (1322a) der Seitenwand des ersten Nutkörpers (1312) nähert oder daran anliegt, und wobei die Wirkkraft, welche mittels des ersten Kontaktabschnitts (133a) auf die äußere Schlauchwand (21) des Katheters (2) erzeugt wird, erhöht wird, wobei unterdessen der zweite Kontaktabschnitt (133b) die äußere Schlauchwand (21) des Katheters (2) berührt und die Wirkkraft erhöht, welche auf die äußere Schlauchwand (21) des Katheters (2) erzeugt wird, so dass die Bewegung des Katheters (2) relativ zu der Positionierungsführungsnut (12) mittels des ersten Kontaktabschnitts (133a) und des zweiten Kontaktabschnitts (133b) beschränkt wird, und wobei die Selbstarretierung-Einheit (132) von dem Freigabe-Zustand aus zu dem Arretierung-Zustand geschaltet wird.

7. Katheter-Positionierung-Vorrichtung gemäß Anspruch 1, **dadurch gekennzeichnet, dass** die Selbstarretierung-Module (13, 130a-130c) ferner ein Substrat (134) aufweisen, und die Selbstarretierung-Einheit (132) mittels Biegens geformt ist und sich von dem Körper des Substrats (134) aus erstreckt,
wobei die V-Nut (131) ferner mit einem oberen Ende (1311), einer Seitenwand des ersten Nutkörpers (1312) und einer Seitenwand des zweiten Nutkörpers (1313) versehen ist, welche sich von dem oberen Ende (1311) aus erstrecken, und wobei das obere Ende (1311) ferner mit einem Spalt versehen ist, und wobei die Seitenwand des ersten Nutkörpers (1312) senkrecht zu der Achse des Katheters (2) ist, welcher in der Positionierungsführungsnut (12) angeordnet ist,
wobei das Substrat (134) an der Basis (11) angeordnet ist und an einer Außenseite der V-Nut (131) positioniert ist, wobei die Selbstarretierung-Einheit (132), welche gebogen ist und sich von dem Substrat (134) aus erstreckt, durch den Spalt hindurchgehen kann, um sich von der Außenseite der V-Nut (131) aus hin zu einem inneren Teil der V-Nut (131) zu erstrecken, und wobei sich die Selbstarretierung-Einheit (132) in einem Bewegung-Abschnitt, welcher mittels der Seitenwand des ersten Nutkörpers (1312) und der Seitenwand des zweiten Nutkörpers (1313) definiert ist, hin und her dreht, und wobei der Kontaktabschnitt (133, 133a, 133b), welcher an dem ersten Endabschnitt der Selbstarretierung-Einheit (132) angeordnet ist, geringfügig aus der V-Nut (131) herausragt und in Kontakt mit der äußeren Schlauchwand (21) des Katheters (2) steht, welcher in der Positionierungsführungsnut (12) angeordnet ist,
wobei, wenn der Katheter (2) an der vordefinierten Position in der Positionierungsführungsnut (12) positioniert ist, sich die Selbstarretierung-Einheit (132) der Seitenwand des zweiten Nutkörpers (1313) nähert oder an der Seitenwand des zweiten Nutkörpers (1313) anliegt, um in dem Freigabe-Zustand zu sein, und das Substrat (134) nicht verformt wird, wobei, wenn sich der Katheter (2) relativ zu der Positionierungsführungsnut (12) bewegt, um von der vordefinierten Position abzuweichen, um sich in der abgewichenen Position zu befinden, sich die Selbstarretierung-Einheit (132) von der Seitenwand des zweiten Nutkörpers (1313) aus zu der Richtung der Seitenwand des ersten Nutkörpers (1312) dreht, um sich der Seitenwand des ersten Nutkörpers (1312) zu nähern oder an der Seitenwand des ersten Nutkörpers (1312) anzuliegen, so dass die Selbstarretierung-Einheit (132) von dem Freigabe-Zustand aus zu dem Arretierung-Zustand geschaltet wird, und das Substrat (134) verformt wird,
wobei, wenn der Katheter (2) von der abgewichenen Position aus wieder an die vordefinierte Position gebracht wird, das verformte Substrat (134) eine Sich-Selbst-Rückstellende-Wirkkraft für die Selbstarretierung-Einheit (132) bereitstellt, so dass sich die Selbstarretierung-Einheit (132) von der Seitenwand des ersten Nutkörpers (1312) aus zu der Richtung der Seitenwand des zweiten Nutkörpers (1313) dreht, um sich der Seitenwand des zweiten Nutkörpers (1313) zu nähern oder daran anzuliegen, und wobei die Selbstarretierung-Module (13, 130a-130c) automatisch in den Freigabe-Zustand zurückgebracht werden.

8. Katheter-Positionierung-Vorrichtung gemäß Anspruch 1, **dadurch gekennzeichnet, dass** die Form des Kontaktabschnitts (133, 133a, 133b) an die Form der äußeren Schlauchwand (21) des Katheters (2) angepasst ist, wobei der Katheter (2) eine kreisförmige Struktur ist, wobei der Kontaktabschnitt (133, 133a, 133b) eine bogenförmige Struktur ist, und wobei der Kontaktabschnitt (133, 133a, 133b) ferner ein erstes Ende (1331), welches an einem Nutboden der Positionierungsführungsnut (12) positioniert ist, und ein zweites Ende (1332) hat, welches an einer Aussparung der Positionierungsführungsnut (12) positioniert ist, wobei das zweite Ende (1332) in der Länge geringfügig kürzer als das erste Ende (1331) ist, um den Katheter (2) in der Positionierungsführungsnut (12) zu befestigen.

9. Katheter-Positionierung-Vorrichtung gemäß Anspruch 8, **dadurch gekennzeichnet, dass**, wenn sich die Selbstarretierung-Einheit (132) in dem Arretierung-Zustand befindet, der Bogenflächenabschnitt (1330) in der bogenförmigen Struktur des Kontaktabschnitts (133, 133a, 133b) eine Wirkkraft auf die äußere Schlauchwand (21) des Katheters (2) in einer dritten Richtung erzeugt, und wobei gleichzeitig das erste Ende (1331) und das zweite Ende (1332) des Kontaktabschnitts (133, 133a, 133b) in jeweils zugeordneter Weise eine Wirkkraft auf die äußere Schlauchwand (21) des Katheters (2) in einer vierten Richtung erzeugen, welche senkrecht zu der dritten Richtung ist.

10. Katheter-Positionierung-Vorrichtung gemäß Anspruch 1, **dadurch gekennzeichnet, dass** die Form des Kontaktabschnitts (133, 133a, 133b) an die Form der äußeren Schlauchwand (21) des Katheters (2) angepasst ist, wobei die Selbstarretierung-Module (13, 130a-130c) paarweise oder in einer versetzten Weise an gegenüberliegenden Seiten der Positionierungsführungsnut (12) angeordnet sind.

11. Katheter-Positionierung-Vorrichtung gemäß Anspruch 1, **dadurch gekennzeichnet, dass** die Positionierungsführungsnut (12) ferner einen Begrenzungsabschnitt (121) zum Begrenzen der Positionierung des Katheters (2) in der Positionierungsführungsnut (12) hat, wobei der Begrenzungsabschnitt (121) ein wellenförmiger Nutrandbereich ist, welcher an einer Aussparung der Positionierungsführungsnut (12) bereitgestellt ist.

12. Katheter-Positionierung-Vorrichtung gemäß Anspruch 1, **dadurch gekennzeichnet, dass** ferner mehrere Hilfspositionierung-Einheiten (122) in der Positionierungsführungsnut (12) bereitgestellt sind zum Hilfspositionieren des Katheters (2) an der vordefinierten Position in der Positionierungsführungsnut (12) .

13. Katheter-Positionierung-Vorrichtung gemäß Anspruch 1, **dadurch gekennzeichnet, dass** die Katheter-Positionierung-Vorrichtung (10) ferner eine Position-Anpass-Vorrichtung aufweist zum Bereitstellen einer Anpassung bezüglich einer Position, an welcher die Selbstarretierung-Module (13, 130a-130c) an der Basis (11) angeordnet sind.

14. Katheter-Positionierung-Vorrichtung gemäß Anspruch 1, **dadurch gekennzeichnet, dass**, wenn sich der Katheter (2) zu einer ersten Richtung relativ zu der Positionierungsführungsnut (12) hin bewegt, um von der vordefinierten Position abzuweichen, ein Teil von den Selbstarretierung-Modulen (13, 130a-130c) automatisch von dem Freigabe-Zustand aus zu dem Arretierung-Zustand geschaltet wird, um den Katheter (2) darin zu beschränken, sich relativ zu der Positionierungsführungsnut (12) in der ersten Richtung zu bewegen, und
wobei, wenn sich der Katheter (2) relativ zu der Positionierungsführungsnut (12) in einer zweiten Richtung, welche entgegengesetzt zu der ersten Richtung ist, bewegt, um von der vordefinierten Position abzuweichen, der andere Teil von den Selbstarretierung-Modulen (13, 130a-130c) automatisch von dem Freigabe-Zustand aus zu dem Arretierung-Zustand geschaltet wird, um den Katheter (2) darin zu beschränken, sich relativ zu der Positionierungsführungsnut (12) in der zweiten Richtung zu bewegen.

## Revendications

1. Dispositif de positionnement de cathéter (10) pour positionner un cathéter (2) sur un corps, comprenant :
une base (11) disposée de manière fixe sur le corps ;
une rainure de guidage de positionnement (12) disposée sur la base (11) et configurée pour accueillir le cathéter (2) ; et
un pluralité de modules autobloquants (13, 130a-130c) disposés sur la base (11) et positionnés sur deux côtés opposés de la rainure de guidage de positionnement (12),
**caractérisé en ce que** chaque module autobloquant (13, 130a-130c) comprend :
une rainure en V (131) ; et
une unité autobloquante (132) disposée de manière mobile dans la rainure en V (131), où une première partie terminale de l'unité autobloquante (132) est pourvue d'une partie de contact configurée pour être en contact avec une paroi de tuyau externe (21) du cathéter (2) disposé dans la rainure de guidage de positionnement (12), et l'unité autobloquante (132) se déplace dans la rainure en V (131) pour commuter entre un état libéré et un état bloqué ;
dans lequel, lorsque le cathéter (2) est positionné à une position prédéfinie dans la rainure de guidage de positionnement (12), chaque module autobloquant (13, 130a-130c) est à l'état libéré ; lorsque le cathéter (2) se déplace par rapport à la rainure de guidage de positionnement (12) pour s'écarter de la position prédéfinie, l'unité autobloquante (132) est verrouillée dans la rainure en V (131) par la partie de contact et la paroi de tuyau externe (21) du cathéter (2), et l'unité autobloquante (132) est commutée automatiquement de l'état libéré à l'état bloqué, de sorte que le mouvement du cathéter (2) par rapport à la rainure de guidage de positionnement (12) est limité par la coordination mutuelle des modules autobloquants (13, 130a-130c) qui sont disposés sur les deux côtés opposés de la rainure de guidage de positionnement (12) et qui sont en état bloqué.

2. Dispositif de positionnement de cathéter selon la revendication 1, **caractérisé en ce que** dans les modules autobloquants (13, 130a-130c),
la rainure en V (131) est également pourvue d'une extrémité supérieure (1311), d'une paroi latérale d'un premier corps de rainure (1312) et d'une paroi latérale d'un deuxième corps de rainure (1313) qui s'étendent à partir de l'extrémité supérieure (1311), et la paroi latérale du premier corps de rainure (1312) et la paroi latérale du deuxième corps de rainure (1313) sont configurées pour définir une section mobile dans la rainure en V (131) ;
l'unité autobloquante (132) comprend en outre une deuxième partie terminale (1321) opposée à la première partie terminale, et une paroi latérale de l'unité autobloquante (1322, 1322a, 1322b) s'étendant de la deuxième partie terminale (1321) à la première partie terminale, où la deuxième partie terminale (1321) de l'unité autobloquante (132) est positionnée dans l'extrémité supérieure (1311) de la rainure en V (131), et la longueur de la paroi latérale de l'unité autobloquante (1322, 1322a, 1322b) est légèrement supérieure à la profondeur de la rainure en V (131), de sorte que la partie de contact (133, 133a, 133b) disposée au niveau de la première partie terminale dépasse légèrement de la rainure en V (131) de manière à être en contact avec la paroi de tuyau externe (21) du cathéter (2) disposé dans la rainure de guidage de positionnement (12) ; et l'unité autobloquante (132) tourne en va-et-vient dans la section mobile de la rainure en V (131) en prenant la deuxième partie terminale (1321) comme extrémité d'arbre, et l'unité autobloquante (132) est commutée entre l'état libéré et l'état bloqué ;
dans lequel, lorsque l'unité autobloquante (132) est à l'état libéré, la partie de contact (133, 133a, 133b) de l'unité autobloquante (132) entre en contact avec la paroi de tuyau externe (21) du cathéter (2) ; lorsque l'unité autobloquante (132) est en état bloqué, une force d'action générée par la partie de contact (133, 133a, 133b) sur la paroi de tuyau externe (21) du cathéter (2) est augmentée, de sorte que la force de frottement générée entre la partie de contact (133, 133a, 133b) et la paroi de tuyau externe (21) est augmentée, et le mouvement du cathéter (2) par rapport à la rainure de guidage de positionnement (12) est limité par la force de frottement suffisante générée entre la partie de contact (133, 133a, 133b) et la paroi de tuyau externe (21).

3. Dispositif de positionnement de cathéter selon la revendication 2, **caractérisé en ce que** l'unité autobloquante (132) est une structure en feuille, la paroi latérale du premier corps de rainure (1312) de la rainure en V (131) est perpendiculaire à un axe du cathéter (2) disposé dans la rainure de guidage de positionnement (12), et l'unité autobloquante (132) a une fonction autobloquante unidirectionnelle ;
dans lequel, lorsque le cathéter (2) est positionné à la position prédéfinie dans la rainure de guidage de positionnement (12), la paroi latérale de l'unité autobloquante (1322, 1322a, 1322b) s'approche de la paroi latérale du deuxième corps de rainure (1313) ou vient en butée contre la paroi latérale du deuxième corps de rainure (1313), la partie de contact (133, 133a, 133b) de l'unité autobloquante (132) entre en contact avec la paroi de tuyau externe (21) du cathéter (2), et l'unité autobloquante (132) est à l'état libéré ;
lorsque le cathéter (2) se déplace dans une première direction par rapport à la rainure de guidage de positionnement (12) pour s'écarter de la position prédéfinie, la paroi latérale de l'unité autobloquante (1322, 1322a, 1322b) tourne de la paroi latérale du deuxième corps de rainure (1313) vers la direction de la paroi latérale du premier corps de rainure (1312) de manière à s'approcher de la paroi latérale du premier corps de rainure (1312) ou à venir en butée contre celle-ci ; et la force d'action générée par la partie de contact (133, 133a, 133b) sur la paroi de tuyau externe (21) du cathéter (2) est augmentée, et l'unité autobloquante (132) est commutée de l'état libéré à l'état bloqué.

4. Dispositif de positionnement de cathéter selon la revendication 2, **caractérisé en ce que** l'unité autobloquante (132) est une structure en feuille, la rainure en V (131) est en outre pourvue d'une surface centrale de référence du corps de rainure (C1) ayant un angle inclus égal avec la paroi latérale du premier corps de rainure (1312) et la paroi latérale du deuxième corps de rainure (1313), la surface centrale de référence du corps de rainure (C1) est perpendiculaire à un axe du cathéter (2) disposé dans la rainure de guidage de positionnement (12), et l'unité autobloquante (132) a une fonction d'autoblocage dans les deux directions ;
dans lequel, lorsque le cathéter (2) est positionné à la position prédéfinie dans la rainure de guidage de positionnement (12), la paroi latérale de l'unité autobloquante (1322, 1322a, 1322b) s'approche de la paroi latérale du premier corps de rainure (1312) ou de la paroi latérale du deuxième corps de rainure (1313) ou vient en butée contre la paroi latérale du premier corps de rainure (1312) ou la paroi latérale du deuxième corps de rainure (1313), la partie de contact (133, 133a, 133b) de l'unité autobloquante (132) entre en contact avec la paroi de tuyau externe (21) du cathéter (2), et l'unité autobloquante (132) est à l'état libéré ;
lorsque le cathéter (2) se déplace dans une première direction par rapport à la rainure de guidage de positionnement (12) ou dans une deuxième direction opposée à la première direction par rapport à la rainure de guidage de positionnement (12) pour s'écarter de la position prédéfinie, la paroi latérale de l'unité autobloquante (1322, 1322a, 1322b) tourne de la paroi latérale du premier corps de rainure (1312) ou de la paroi latérale du deuxième corps de rainure (1313) en direction de la surface centrale de référence du corps de rainure (C1) de manière à s'approcher de la surface centrale de référence du corps de rainure (C1) ; et la force d'action générée par la partie de contact (133, 133a, 133b) sur la paroi de tuyau externe (21) du cathéter (2) est augmentée, et l'unité autobloquante (132) est commutée de l'état libéré à l'état bloqué.

5. Dispositif de positionnement de cathéter selon la revendication 2, **caractérisé en ce que** l'unité autobloquante (132) est une structure de feuille en forme de V, et a une deuxième partie terminale (1321), deux premières parties terminales, une paroi latérale de la première unité autobloquante (1322a) et une paroi latérale de la deuxième unité autobloquante (1322b) s'étendant respectivement de ladite une deuxième partie terminale (1321) aux deux premières parties terminales, et une première partie de contact (133a) et une deuxième partie de contact (133b) respectivement disposées sur les deux premières parties terminales, et une surface centrale de référence de l'unité autobloquante (C2) ayant un angle inclus égal avec la paroi latérale de la première unité autobloquante (1322a) et la paroi latérale de la deuxième unité autobloquante (1322b) respectivement ; et la rainure en V (131) est également pourvue d'une surface centrale de référence du corps de rainure (C1) ayant un angle inclus égal avec la paroi latérale du premier corps de rainure (1312) et la paroi latérale du deuxième corps de rainure (1313) respectivement, la surface centrale de référence du corps de rainure (C1) est perpendiculaire à l'axe du cathéter (2) disposé dans la rainure de guidage de positionnement (12), un angle d'ouverture de l'unité autobloquante (132) est légèrement inférieur à celui de la rainure en V (131), et l'unité autobloquante (132) a une fonction d'autoblocage dans les deux directions ;
dans lequel, lorsque le cathéter (2) est positionné à la position prédéfinie dans la rainure de guidage de positionnement (12), la surface centrale de référence de l'unité autobloquante (C2) s'approche de ou coïncide avec la surface centrale de référence du corps de rainure (C1), la première partie de contact (133a) et la deuxième partie de contact (133b) de l'unité autobloquante (132) entrent en contact avec la paroi de tuyau externe (21) du cathéter (2), et l'unité autobloquante (132) est à l'état libéré ;
lorsque le cathéter (2) se déplace dans une première direction par rapport à la rainure de guidage de positionnement (12) pour s'écarter de la position prédéfinie, la surface centrale de référence de l'unité autobloquante (C2) tourne à partir de la surface centrale de référence du corps de rainure (C1) en direction de la paroi latérale du premier corps de rainure (1312), de sorte que la paroi latérale de l'unité autobloquante (1322, 1322a, 1322b) s'approche de la paroi latérale du premier corps de rainure (1312) de la rainure en V (131) ou vient en butée contre celle-ci ; la force d'action générée par la deuxième partie de contact (133b) sur la paroi de tuyau externe (21) du cathéter (2) est augmentée, et l'unité autobloquante (132) est commutée de l'état libéré à l'état bloqué ; ou lorsque le cathéter (2) se déplace dans une deuxième direction opposée à la première direction par rapport à la rainure de guidage de positionnement (12) pour s'écarter de la position prédéfinie, la surface centrale de référence de l'unité autobloquante (C2) tourne de la surface centrale de référence du corps de rainure (C1) en direction de la paroi latérale du deuxième corps de rainure (1313), de sorte que la paroi latérale de la deuxième unité autobloquante (1322b) s'approche de ou vient en butée contre la paroi latérale du deuxième corps de rainure (1313) de la rainure en V (131) ; et la force d'action générée par la première partie de contact (133a) sur la paroi de tuyau externe (21) du cathéter (2) est augmentée, et l'unité autobloquante (132) est commutée de l'état libéré à l'état bloqué.

6. Dispositif de positionnement de cathéter selon la revendication 2, **caractérisé en ce que** l'unité autobloquante (132) est une structure de feuille en forme de V, et a une deuxième partie terminale (1321), deux premières parties terminales, une paroi latérale de la première unité autobloquante (1322a) et une paroi latérale de la deuxième unité autobloquante (1322b) s'étendant respectivement de la deuxième partie terminale (1321) aux deux premières parties terminales, et une première partie de contact (133a) et une deuxième partie de contact (133b) respectivement disposées sur les deux premières parties terminales ; la paroi latérale du premier corps de rainure (1312) de la rainure en V (131) est perpendiculaire à un axe du cathéter (2) disposé dans la rainure de guidage de positionnement (12), l'angle d'ouverture de l'unité autobloquante (132) est légèrement inférieur à celui de la rainure en V (131), et l'unité autobloquante (132) a une fonction d'autoblocage unidirectionnelle ;
dans lequel, lorsque le cathéter (2) est positionné à la position prédéfinie dans la rainure de guidage de positionnement (12), la paroi latérale de la deuxième unité autobloquante (1322b) s'approche de la paroi latérale du deuxième corps de rainure (1313) ou vient en butée contre celle-ci, la première partie de contact (133a) entre en contact avec la paroi de tuyau externe (21) du cathéter (2), et l'unité autobloquante (132) est à l'état libéré ;
lorsque le cathéter (2) se déplace dans une première direction par rapport à la rainure de guidage de positionnement (12) pour s'écarter de la position prédéfinie, l'unité autobloquante (132) tourne de la paroi latérale du deuxième corps de rainure (1313) en direction de la paroi latérale du premier corps de rainure (1312), de sorte que la paroi latérale de la première unité autobloquante (1322a) s'approche de la paroi latérale du premier corps de rainure (1312) ou vient en butée contre celle-ci, et la force d'action générée par la première partie de contact (133a) sur la paroi de tuyau externe (21) du cathéter (2) est augmentée ; pendant ce temps, la deuxième partie de contact (133b) entre en contact avec la paroi de tuyau externe (21) du cathéter (2) et augmente la force d'action générée sur la paroi de tuyau externe (21) du cathéter (2), de sorte que le mouvement du cathéter (2) par rapport à la rainure de guidage de positionnement (12) est limité par la première partie de contact (133a) et la deuxième partie de contact (133b), et l'unité autobloquante (132) est commutée de l'état libéré à l'état bloqué.

7. Dispositif de positionnement de cathéter selon la revendication 1, **caractérisé en ce que** les modules autobloquants (13, 130a-130c) comprennent en outre un substrat (134), et l'unité autobloquante (132) est formée par pliage et s'étend à partir du corps du substrat (134) ;
dans lequel la rainure en V (131) est en outre pourvue d'une extrémité supérieure (1311), d'une paroi latérale du premier corps de rainure (1312) et d'une paroi latérale du deuxième corps de rainure (1313) qui s'étendent à partir de l'extrémité supérieure (1311), et l'extrémité supérieure (1311) est en outre pourvue d'un espace, et la paroi latérale du premier corps de rainure (1312) est perpendiculaire à l'axe du cathéter (2) disposé dans la rainure de guidage de positionnement (12) ;
le substrat (134) est disposé sur la base (11) et positionné sur un côté extérieur de la rainure en V (131), l'unité autobloquante (132) pliée et étendue à partir du substrat (134) peut passer à travers l'espace pour s'étendre du côté extérieur de la rainure en V (131) à une partie intérieure de la rainure en V (131), et l'unité autobloquante (132) tourne en va-et-vient dans une section mobile définie par la paroi latérale du premier corps de rainure (1312) et la paroi latérale du deuxième corps de rainure (1313) ; et la partie de contact (133, 133a, 133b) disposée sur la première partie terminale de l'unité autobloquante (132) dépasse légèrement de la rainure en V (131) et est en contact avec la paroi de tuyau externe (21) du cathéter (2) disposé dans la rainure de guidage de positionnement (12) ;
dans lequel, lorsque le cathéter (2) est positionné à la position prédéfinie dans la rainure de guidage de positionnement (12), l'unité autobloquante (132) s'approche de la paroi latérale du deuxième corps de rainure (1313) ou vient en butée contre la paroi latérale du deuxième corps de rainure (1313) pour être à l'état libéré, et le substrat (134) n'est pas déformé ; lorsque le cathéter (2) se déplace par rapport à la rainure de guidage de positionnement (12) pour s'écarter de la position prédéfinie pour se trouver dans la position écartée, l'unité autobloquante (132) tourne depuis la paroi latérale du deuxième corps de rainure (1313) en direction de la paroi latérale du premier corps de rainure (1312) de manière à s'approcher de la paroi latérale du premier corps de rainure (1312) ou à venir en butée contre la paroi latérale du premier corps de rainure (1312), de sorte que l'unité autobloquante (132) est commutée de l'état libéré à l'état bloqué, et le substrat (134) est déformé ;
dans lequel, lorsque le cathéter (2) est ramené pour être positionné à la position prédéfinie à partir de la position écartée, le substrat déformé (134) fournit une force d'action d'auto-réinitialisation pour l'unité autobloquante (132), de sorte que l'unité autobloquante (132) tourne de la paroi latérale du premier corps de rainure (1312) en direction de la paroi latérale du deuxième corps de rainure (1313) de manière à s'approcher ou à venir en butée contre la paroi latérale du deuxième corps de rainure (1313), et les modules autobloquants (13, 130a-130c) sont automatiquement ramenés à l'état libéré.

8. Dispositif de positionnement de cathéter selon la revendication 1, **caractérisé en ce que** la forme de la partie de contact (133, 133a, 133b) est adaptée à la forme de la paroi de tuyau externe (21) du cathéter (2), le cathéter (2) est une structure circulaire, la partie de contact (133, 133a, 133b) est une structure en forme d'arc, et la partie de contact (133, 133a, 133b) a en outre une première extrémité (1331) positionnée à un fond de rainure de la rainure de guidage de positionnement (12) et une deuxième extrémité (1332) positionnée au niveau d'une encoche de la rainure de guidage de positionnement (12), la deuxième extrémité (1332) étant légèrement plus courte que la première extrémité (1331) afin de monter le cathéter (2) dans la rainure du guide de positionnement (12).

9. Dispositif de positionnement de cathéter selon la revendication 8, **caractérisé en ce que**, lorsque l'unité autobloquante (132) est en état bloqué, la partie de surface en arc (1330) dans la structure en forme d'arc de la partie de contact (133, 133a, 133b) génère une force d'action sur la paroi de tuyau externe (21) du cathéter (2) dans une troisième direction ; et simultanément, la première extrémité (1331) et la deuxième extrémité (1332) de la partie de contact (133, 133a, 133b) génèrent respectivement une force d'action sur la paroi de tuyau externe (21) du cathéter (2) dans une quatrième direction perpendiculaire à la troisième direction.

10. Dispositif de positionnement de cathéter selon la revendication 1, **caractérisé en ce que** la forme de la partie de contact (133, 133a, 133b) est adaptée à la forme de la paroi de tuyau externe (21) du cathéter (2), les modules autobloquants (13, 130a-130c) sont disposés par paires ou en quinconce sur des côtés opposés de la rainure de guidage de positionnement (12).

11. Dispositif de positionnement de cathéter selon la revendication 1, **caractérisé en ce que** la rainure de guidage de positionnement (12) comporte en outre une partie limite (121) destinée à restreindre le positionnement du cathéter (2) dans la rainure de guidage de positionnement (12), la partie limite (121) étant un bord de rainure ondulé prévu au niveau d'une encoche de la rainure de guidage de positionnement (12).

12. Dispositif de positionnement de cathéter selon la revendication 1, **caractérisé en ce qu'**une pluralité d'unités de positionnement auxiliaires (122) sont en outre prévues dans la rainure de guidage de positionnement (12) pour le positionnement auxiliaire du cathéter (2) à la position prédéfinie dans la rainure de guidage de positionnement (12).

13. Dispositif de positionnement de cathéter selon la revendication 1, **caractérisé en ce que** le dispositif de positionnement de cathéter (10) comprend en outre un ensemble d'ajustement de position pour fournir un ajustement à une position à laquelle les modules autobloquants (13, 130a-130c) sont disposés sur la base (11).

14. Dispositif de positionnement de cathéter selon la revendication 1, **caractérisé en ce que**, lorsque le cathéter (2) se déplace dans une première direction par rapport à la rainure de guidage de positionnement (12) pour s'écarter de la position prédéfinie, une partie des modules autobloquants (13, 130a-130c) est commutée automatiquement de l'état libéré à l'état bloqué de manière à limiter le déplacement du cathéter (2) par rapport à la rainure de guidage de positionnement (12) dans la première direction, et
lorsque le cathéter (2) se déplace par rapport à la rainure de guidage de positionnement (12) dans une deuxième direction opposée à la première direction pour s'écarter de la position prédéfinie, l'autre partie des modules autobloquants (13, 130a-130c) est commutée automatiquement de l'état libéré à l'état bloqué de manière à limiter le déplacement du cathéter (2) par rapport à la rainure de guidage de positionnement (12) dans la deuxième direction.
